# EUROPEAN PATENT APPLICATION

(11) **EP 2 402 430 A1**
(43) Date of publication of application: **04.01.2012**
(21) Application number: 10745996.8
(22) Date of filing: 25.02.2010
(51) Int. Cl.: C12N 1/20

(54) **PRODUCTION METHOD FOR SELECTIVE MEDIUM AND USE THEREOF**

(30) Priority: 25.02.2009 JP 2009042108
(71) Applicant: The University of Tokyo, Bunkyo-Ku Tokyo 113-8654 (JP)
(72) Inventor: NAMBA, Shigetou, Tokyo 113-8654 (JP); HAMAMOTO, Hiroshi, Tokyo 113-8654 (JP); KAWANISHI, Takeshi, Tokyo 113-8654 (JP)
(74) Representative: Polypatent
(86) International application number: PCT/JP2010/001290
(87) International publication number: WO 2010/098113

(57) **Abstract**

An object to be solved by the present invention is to provide a method for producing a selective medium that allows a target microorganism to predominantly proliferate in the simultaneous presence of many different microorganism according to specific criteria. The object is solved by a method for producing a selective medium for a microorganism, which comprises the steps of: selecting at least one carbon source assimilable by a microorganism and/or at least one antibiotic to which the microorganism has resistance based on biological information about the microorganism; and mixing the selected carbon source and/or antibiotic with basic medium components.

## Description

### Technical Field

The present invention relates to a method for producing a selective medium for a microorganism which comprises selecting components that allow a microorganism to predominantly proliferate based on biological information about the microorganism and mixing the selected components with basic medium components. Further, the present invention relates to a selective medium for a microorganism produced by the method and a kit for detecting microorganisms that comprises the selective medium.

### Background Art

Animal/plant pathogens are spread through airborne transmission, contact transmission, rainfall transmission and the like and cause serious damage to animals/plants. For instance, examples of plant pathogens include seed-borne pathogens. There is a tendency that contamination with seed-borne pathogens spreads even through seed lots with very low contamination rates, causing expansion of damage. Particularly in the case of cooperative seedling raising, grafting cultivation or the like for cultivation of a number of seedlings, such contamination would cause serious damage.

In order to avoid such seed-borne diseases, each seed lot is examined whether or not seeds have pathogens. Examples of a method for examining contaminated seeds are grow-out tests and sweat-box-grow-out tests wherein seeds are sown on culture soil in a greenhouse or a plastic box and evaluation is carried out based on symptoms observed in germinated seedlings. These methods are advantageous in that pathogens can be identified based on symptoms. However, it is necessary for such methods to be conducted in a facility having a wide space where temperature and humidity are controlled at constant levels appropriate for the growth of seed-borne pathogens depending on the number of test seeds, Further, according to such methods, the test seed germination rate is not always 100%. Therefore, it is probable that no germination of contaminated seeds would cause a test lot to be evaluated as false negative, which in turn indicates no contamination of the test lot with pathogens. In addition, there have been few examples of examination by the above methods conducted in Japan. Therefore, in practice, overseas examination facilities have been assigned to conduct examination by the methods.

As a known method for detecting contaminated seeds other than the above methods, there is a colony detection method wherein the presence of a pathogen is detected as colony. The colony detection method is usually carried out in the following manner. Seeds are soaked in a solution, followed by agitation and washing. Then, the obtained seed washing liquid is directly (or first precultured and then) smeared on a selective medium that allows a target pathogen to more predominantly grow than other pathogens, followed by incubation under conditions appropriate for the pathogens. For example, as selective media used for such method, selective media in which the following bacteria can specifically proliferate have been known to the public: *Burkholderia glumae, Acidovorax avenue* subsp. *avenue, Agrobacterium tumefaciens, Burkholderia caryophylli, Erwinia carotovara* subsp. *carotovora, Ralstonia solanacearum,* and *Xanthomonas campestris* pv. *campestris* (Non-Patent Documents 1 to 7).

### Prior Art Documents

### Non-Patent Documents

Non-Patent Document 1: Kawaradani M, Okada K, Kusakari S, (2000), Journal of General Plant Pathology 66, 234-237
Non-Patent Document 2: Takashi Shirakawa, Masao Aizawa, Yukiko Komiya, Kazuo Abiko (2000), Development of selective medium for separation/detection of Acidovorax avenae subsp. citrulli from seeds and plants, Japanese Journal of Phytopathology, 66, 132
Non-Patent Document 3: Kado, C. I. and M. G. Heskett. (1970), Phytopathology 60: 969-976.
Non-Patent Document 4: Nobuo Aono and Kunihiko Kato (1979), Examination of selective medium for bacterial wilt of carnation, Bulletin of Kanagawa Horticultural Experiment Station, 26, 84-89
Non-Patent Document 5: Hiroyuki Tsuyama (1962), Study on soft rot of Chinese cabbage (napa cabbage), Bull. Inst. Agr. Res. Tohoku Univ., 13, 221-345
Non-Patent Document 6: Hidenori Hara and Kuniaki Ono (1982), Study on etiology and mechanism of bacterial wilt of tobacco, Bulletin of Okayama Tobacco Experiment Station, 42, 127-138
Non-Patent Document 7: Chun WWC and Alvarez AM (1983), Plant Disease 67, 632-635

### Disclosure of the Invention

### Object to be Solved by the Invention

Colony detection methods have many advantageous features over the grow-out tests and sweat-box-grow-out tests. For example, the space necessary for selective medium culture can be made smaller than the space necessary for seed germination. Contaminated seeds can be detected regardless of the test seed germination rate. In addition, the methods can be carried out in a convenient manner at domestic facilities. Further, according to the colony detection methods, the degree of seed contamination can be determined based on the number of colonies of a pathogen.

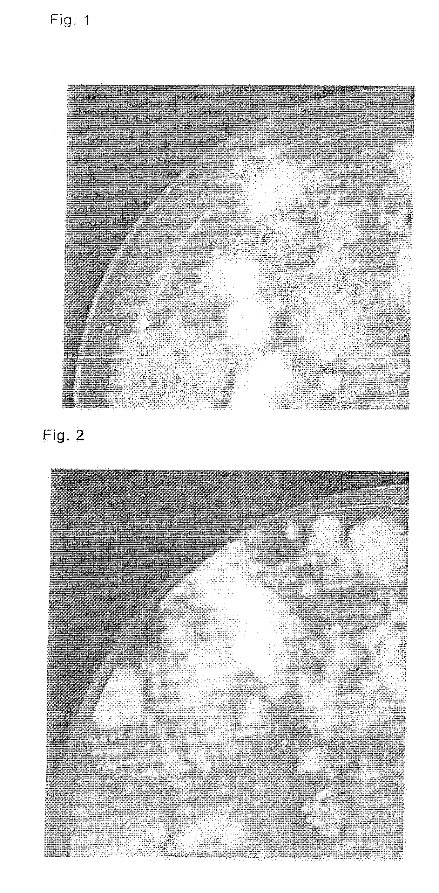

However, according to the conventional colony detection methods, it is difficult to detect a pathogen and particularly the degree of contamination with a pathogen. This is because, as shown in figs. 1 to 7, it is necessary to select colonies of a target pathogen from among colonies of a variety of microorganisms.

One possible reason that causes the above problems regarding the conventional colony detection methods is that conventional selective media have poor selectivity of target microorganisms such as pathogens. In particular, a selective medium that allows a target microorganism to predominantly proliferate in the simultaneous presence of many different microorganisms and a method for producing such selective medium have not been known to the public. The composition of a conventionally used selective medium is determined depending on the type of microorganism according to so-called empirical rules. However, a method for producing a selective medium in accordance with specific criteria regarding properties of microorganisms and the like also has not been known to the public.

Therefore, an object of the present invention is to provide a method for producing a selective medium that allows a target microorganism to predominantly proliferate in the simultaneous presence of many different microorganisms according to specific criteria. Further, another object of the present invention is to provide a selective medium which is produced by the production method and a kit for detecting microorganisms which comprises the selective medium.

### Means for Solving Object

As a result of intensive studies conducted to achieve the above object, the present inventors found that a microorganism can be predominantly cultured by using a selective medium which is produced by a method comprising: selecting a carbon source which can be predominantly assimilated by a target microorganism and/or an antibiotic to which the microorganism has resistance based on biological information about the target microorganism through bioinformatics; and mixing the selected carbon source and/or the antibiotic with basic medium components. This has led to the completion of the present invention. The present invention provides a method for producing a selective medium for a microorganism, which comprises the steps of: selecting at least one carbon source assimilable by a microorganism and/or at least one antibiotic to which the microorganism has resistance based on biological information about the microorganism; and mixing the selected carbon source and/or antibiotic with basic medium components.

Another aspect of the present invention provides a method for producing a selective medium for a microorganism, which comprises the steps of: selecting at least one carbon source assimilable by the microorganism and at least one antibiotic to which the microorganism has resistance based on biological information about the microorganism; and mixing the selected carbon source and antibiotic with basic medium components.

In a preferred embodiment of the method for producing a selective medium for a microorganism according to the present invention, the basic medium components comprise a nitrogen source, a buffer substance, and a mineral substance.

In a preferred embodiment of the method for producing a selective medium for a microorganism according to the present invention, the microorganism is a bacterium or a fungus.

In a preferred embodiment of the method for producing a selective medium for a microorganism according to the present invention, the bacterium is *Acidovorax avenae* subsp. *avenae, Agrobacterium tumefaciens, Burkholderia caryophylli, Burkholderia glumae, Erwinia carotovoru* subsp. *carotovora, Ralstonia solanacearum, Xanthomonas campestris* pv. *campestris,* or *Burkholderia cepacia.*

In a preferred embodiment of the method for producing a selective medium for a microorganism according to the present invention, the biological information based on which the carbon source is selected is information about carbon metabolism of the microorganism.

In a preferred embodiment of the method for producing a selective medium for a microorganism according to the present invention, the biological information based on which the antibiotic is selected is information about the gene of the microorganism.

In a preferred embodiment of the method for producing a selective medium for a microorganism according to the present invention, the gene is an antibiotic-resistant gene or an antibiotic synthesis gene.

In a preferred embodiment of the method for producing a selective medium for a microorganism according to the present invention, at least one carbon source assimilable by the microorganism is at least one carbon source selected from the group consisting of arabinose, mannitol, trehalose, inositol, arginine, aspartic acid, isoleucine, leucine, lysine, methionine, phenylalanine, proline, threonine, glutamic acid, tyrosine, valine, and polygalacturonic acid.

In a preferred embodiment of the method for producing a selective medium for a microorganism according to the present invention, the at least one antibiotic to which the microorganism has resistance is at least one antibiotic selected from the group consisting of ampicillin, chloramphenicol, novobiocin, cetrimide, polymyxin, carbenicillin, ticarcillin, penicillin, neomycin, tyrothricin, streptomycin, and vancomycin.

In a preferred embodiment of the method for producing a selective medium for a microorganism according to the present invention, the selective medium for a microorganism is a complete synthetic medium.

Another aspect of the present invention provides a selective medium for a microorganism, which is produced by the production method according to the present invention.

In a preferred embodiment of the selective medium according to the present invention, the microorganism is *Burkholderia glumae,* arabinose is contained as the carbon source, and at least one antibiotic selected from the group consisting of ampicillin, chloramphenicol, novobiocin, cetrimide, and polymyxin is contained as the antibiotic.

In a preferred embodiment of the selective medium according to the present invention, the microorganism is *Acidovorax avenae* subsp. *avenae,* mannitol is contained as the carbon source, and at least one antibiotic selected from the group consisting of carbenicillin, ticarcillin, penicillin, and neomycin is contained as the antibiotic.

In a preferred embodiment of the selective medium according to the present invention, the microorganism is *Agrobacterium tumefaciens,* mannitol is contained as the carbon source, and at least one antibiotic selected from the group consisting of tyrothricin, cetrimide, and vancomycin is contained as the antibiotic.

In a preferred embodiment of the selective medium according to the present invention, the microorganism is *Burkholderia caryophylli,* sorbitol is contained as the carbon source, and at least one antibiotic selected from the group consisting of tyrothricin, ampicillin, chloramphenicol, and polymyxin is contained as the antibiotic.

In a preferred embodiment of the selective medium according to the present invention, the microorganism is *Erwinia carotovara* subsp. *carotovora,* pectic acid is contained as the carbon source, and at least one antibiotic selected from the group consisting of neomycin, novobiocin, cetrimide, and vancomycin is contained as the antibiotic.

In a preferred embodiment of the selective medium according to the present invention, the microorganism is *Ralstonia solanacearum,* L-glutamic acid is contained as the carbon source, and at least one antibiotic selected from the group consisting of polymyxin, chloramphenicol, ticarcillin, carbenicillin, and vancomycin is contained as the antibiotic.

In a preferred embodiment of the selective medium according to the present invention, the microorganism is *Xanthomonas campestris* pv. *campestris,* mannitol is contained as the carbon source, and at least one antibiotic selected from the group consisting of novobiocin, neomycin, and vancomycin is contained as the antibiotic.

In a preferred embodiment of the selective medium according to the present invention, the microorganism is *Burkholderia cepacia,* arabinose is contained as the carbon source, and at least antibiotic selected from the group consisting of streptomycin and ampicillin is contained as the antibiotic.

In a preferred embodiment of the selective medium according to the present invention, the form is a liquid medium, a solid medium, or a medium-impregnated support.

Another aspect of the present invention provides a kit for detecting a microorganism, which comprises the selective medium according to the present invention.

In a preferred embodiment of the kit according to the present invention the kit is in the form of a stick-type kit or a liquid-type kit.

### Effects of the Invention

According to the production method of the present invention, a selective medium that allows a target microorganism to predominantly proliferate can be produced by inhibiting proliferation of non-desired microorganisms. With the use of the selective medium of the present invention produced by the production method of the present invention, microorganisms such as plant pathogenic microorganisms (e.g., *Acidovorax avenae* subsp. *avenae, Agrobacterium tumefaciens, Burkholderia caryophylli, Burkholderia glumae, Erwinia carotovora* subsp. *carotovora, Ralstonia solanacearum,* and *Xanthomonas campestris* pv. *campestris*) and animal pathogenic microorganisms (e.g., *Burkholderia cepacia*) are allowed to more predominantly proliferate than other microorganism species. A microorganism used as a target microorganism can be a microorganism for which genomic information has been elucidated or a microorganism about which abundant gene or enzyme information is available. When the selective medium of the present invention is a complete synthetic medium, it has improved heat resistance, it can be produced with fewer lot differences, and a risk related to unavailability of starting materials can be reduced compared with a medium containing natural components. With the use of selective medium and the kit of the present invention, it becomes possible to detect microorganism contamination in a sample in an accurate and convenient manner by visually checking color development or turbidity derived from microorganism proliferation. Accordingly, the present invention is highly expected to prevent spread of microorganism contamination. In addition, there is not need to perform DNA extraction or isolation of bacteria when the selective medium or the kit of the present invention is used. Also, PCR equipments and the like are not necessary, unlike the cases of conventionally known methods such as a method for detecting the presence or absence of a target microorganism by PCR amplification of a portion of DNA extracted from a population consisting of a variety of microorganisms with the use of primers specific to the microorganism and a method for detecting the presence or absence of a microorganism through an antigen-antibody reaction using an antibody specific to the microorganism. Therefore, the selective medium or the kit of the present invention enables detection of the presence or absence of a target microorganism in a large amount of a sample in a convenient and rapid manner as compared with the above methods.

### Brief Description of the Drawings

Fig. 1 shows results obtained by culturing *Acidovorax avenue* subsp. *avenae* in the previously reported selective medium.
Fig. 2 shows results obtained by culturing *Agrobacterium tumefaciens* in the previously reported selective medium.
Fig. 3 shows results obtained by culturing *Burkholderia caryophylli* in the previously reported selective medium.
Fig. 4 shows results obtained by culturing *Burkholderia glumae* in the previously reported selective medium.
Fig. 5 shows results obtained by culturing *Erwinia carotovara* subsp. *carotovora* in the previously reported selective medium.
Fig. 6 shows results obtained by culturing *Ralstonia solanacearum* in the previously reported selective medium.
Fig. 7 shows results obtained by culturing *Xanthomonas campestris* pv. *campestris* in the previously reported selective medium.
Fig. 8 shows an example of the structure of the selective medium of the present invention in the form of a medium-impregnated support.
Fig. 9 shows an example of the stick-type kit of the present invention.
Fig. 10 shows the outline of a method for using the kit shown in Fig. 9.
Fig. 11 shows an example of the stick-type kit of the present invention.
Fig. 12 shows results obtained by culturing *Acidovorax avenae* subsp. *avenae* in the selective medium of the present invention.
Fig. 13 shows results obtained by culturing *Agrobacterium tutnefaciens* in the selective medium of the present invention.
Fig. 14 shows results obtained by culturing *Burkholderia caryophylli* in the selective medium of the present invention.
Fig. 15 shows results obtained by culturing *Burkholderia glumae* in the selective medium of the present invention.
Fig. 16 shows results obtained by culturing *Erwinia carotovara* subsp. *carotovora* in the selective medium of the present invention.
Fig. 17 shows results obtained by culturing *Ralstonia solanacearum* in the selective medium of the present invention.
Fig. 18 shows results obtained by culturing *Xanthomonas campestris* pv. *campestris* in the selective medium of the present invention.
Fig. 19 is a chart showing the change in the number of microorganisms during selective culture of *Burkholderia glumae* described in Example 8(4).
Fig. 20 is a chart showing the change in the number of microorganisms during selective culture of *Burkholderia caryophylli* described in Example 8(6).
Fig. 21 shows selective culture results for *Burkholderia caryophylli* obtained in Example 9.
Fig. 22 shows results obtained by culturing *Burkholderia cepacia* in the selective medium produced by OXOID.
Fig. 23 shows results obtained by culturing *Burkholderia cepacia* in the selective medium of the present invention.

### Embodiment for Carrying Out the Invention

The present invention is described below in detail.

The production method of the present invention relates to a method for producing a selective medium for a microorganism, which comprises mixing components that are selected to allow a microorganism to predominantly proliferate based on biological information about the microorganism with basic medium components.

The term "selective medium for a microorganism" refers to a medium that allows a microorganism to be targeted (hereinafter sometimes referred to as a "target microorganism") to predominantly proliferate. The expression "predominantly" used herein means that the number of cells of a target microorganism that is allowed to proliferate on a medium is larger than the total number of cells of other microorganisms. For example, the ratio of the former to the latter is preferably 60:40, more preferably 70:30, further preferably 80:20, furthermore preferably 90:10, and particularly preferably 95:5.

Examples of components selected based on biological information about a microorganism include nutrients required for proliferation of a target microorganism and substances capable of inhibiting proliferation of microorganisms other than a target microorganism, which are preferably carbon sources and antibiotics. According to the production method of the present invention, at least one carbon source and/or at least one antibiotic can be selected.

At least one component selected based on biological information about a microorganism may be used. For instance, any carbon source or antibiotic may be used. However, in order to cause a target microorganism to predominantly proliferate, it is preferable to use at least two types of components selected based on biological information about a microorganism. For instance, it is preferable to select both carbon sources and antibiotics.

Basic medium components include, for example, a nitrogen source, a mineral substance, and a buffer substance to achieve the minimum necessary medium composition for microorganism proliferation. Note that if components selected based on biological information about a microorganism include no carbon source, basic medium components include a carbon source.

A nitrogen source included in basic medium components is not particularly limited as long as it is an inorganic nitrogen-containing compound. However, as a nitrogen source, an ammonium salt or nitrate can be used. Specific examples of a nitrogen source that can be used include ammonium chloride, ammonium sulfate, ammonium nitrate, sodium nitrate, and ammonium phosphate. Note that if an amino acid such as glutamic acid or an organic nitrogen-containing compound such as urea is used as a carbon source, such substance also can be used as a nitrogen source.

A buffer substance included in basic medium components is not particularly limited as long as it is a substance having a buffering function of inhibiting pH variation or a combination of such substances. However, examples of a buffer substance that can be used include substances constituting buffer such as acetic acid buffer, citric acid buffer, boric acid buffer, tartaric acid buffer, Tris buffer, or phosphate buffer saline. Specific examples of a combination of such substances that can be used include: acetic acid and sodium acetate; phosphoric acid and sodium dihydrogen phosphate; sodium dihydrogen phosphate and disodium hydrogen phosphate; potassium dihydrogen phosphate and disodium hydrogen phosphate; citric acid and sodium citrate; Tris(hydroxymethyl)aminomethane and hydrochloric acid; Tris(hydroxymethyl)aminomethane and ethylenediaminetetraacetate; Tris(hydroxymethyl)aminomethane, acetic acid, and ethylenediaminetetraacetate; Tris(hydroxymethyl)aminomethane, boric acid, and ethylenediaminetetraacetate; Tris(hydroxymethyl)aminomethane and sodium chloride; boric acid and sodium chloride; tartaric acid and sodium tartrate. However, a carbon-free compound or a combination of carbon-free compounds is preferable.

A mineral substance included in basic medium components is not particularly limited as long as it is a mineral substance required for microorganism proliferation. However, examples of a mineral substance that can be used include a calcium salt, a magnesium salt, a potassium salt, a sodium salt, a phosphate salt, a manganese salt, a zinc salt, an iron salt, a copper salt, a molybdenum salt, and a cobalt salt. Specific examples of a mineral substance that can be used include potassium dihydrogen phosphate, dipotassium hydrogen phosphate, zinc magnesium, ferrous sulfate, zinc manganese, zinc sulfate, sodium chloride, potassium chloride, and calcium chloride. A mineral substance that should be included in basic medium components can be determined based on, for example, analysis results obtained by component analysis of a dried microorganism. Further, a specific mineral substance is required for metabolism or gene expression depending on components selected based on biological information in some cases. In such cases, it is preferable to adequately add a required mineral substance as a basic medium component so as to allow a microorganism to use components selected based on biological information.

According to the production method of the present invention, vitamins such as biotin and thiamine can be adequately added according to need. Further, in order to facilitate detection of microorganism proliferation, it is preferable to add a staining substance (stain) for coloring proliferated microorganisms. A stain to be used is not particularly limited as long as it is a stain having affinity to a commonly known microorganism. However, when a microorganism is a bacterium, BTB, crystal violet, or the like can be used. In particular, when a microorganism is a gram-negative bacterium, crystal violet is preferably used with an expectation that it would exhibit bactericidal effects on gram-positive bacteria. In addition, according to the production method of the present invention, it is preferable to add antibiotics and the like which inhibit proliferation of microorganisms other than a target microorganism. For example, when a microorganism is a bacterium, it is preferable to add antifungal antibiotics such as cycloheximide to inhibit fungal proliferation. The above vitamins, stains, antibiotics, and the like can be included in basic medium components or substances to be added to basic medium components according to the production method of the present invention. A selective medium obtained in the production method of the present invention can contain substance selected from the group consisting of nitrogen sources, buffer substances, mineral substances, vitamins, stains, antibiotics, and other substances, each substance being one substance or two or more substances.

Biological information about a microorganism can be used without limitation as long as it is biological information related to microorganism. Biological information can be adequately adjusted depending on components to be selected. However, identical or different biological information can be used depending on components to be selected. Examples of biological information about a microorganism include gene information, enzyme information, and each nutrient metabolism information about a microorganism. When a component to be selected is a carbon source, carbon metabolism information is preferable. When a component to be selected is an antibiotic, information about antibiotic-resistant genes and antibiotic synthesis genes is preferable. Biological information about a microorganism can be obtained at the website of a public database created based on microorganism genome information such as KEGG or NCBI.

Microorganisms used herein encompass all prokaryotes and eukaryotes without limitation. Specific examples thereof include bacteria, fungi, protists, and tissues and organs of plants and animals. Preferably, a target microorganism used in the present invention is a microorganism, biological information of which can be obtained using a public database or the like. More preferably, it is a bacterium or fungus. The present invention can be carried out using, as a target microorganism, a bacterium or fungus pathogenic to a plant or animal selected from among bacteria and fungi and specifically from among plant pathogenic bacteria, plant pathogenic fungi, animal pathogenic bacteria, and animal pathogenic fungi. Examples of plant pathogenic bacteria include, but are not limited to, bacteria listed in Table 1 below. In Table 1, the bacteria listed in the left column can cause the corresponding diseases listed in the right column.

**Table 1:**

| Bacterial name | Disease name |
|---|---|
| *Acidovorax venae* subsp. *avenae* | Bacterial brown stripe of rice |
| *Acidovorax avenae* subsp. *cattleyae* | Bacterial brown spot of cattleya |
| *Acidovorax konjaci* | Konnyaku bacterial leaf blight |
| *Agrobacterium rhizogenes* | Hairy root of melon |
| *Agrobacterium tumefaciens* | Crown gall |
| *Burkholderia andropogonis* | Bacterial spot of carnation |
| *Burkholderia caryophylli* | Bacterial wilt of carnation |
| *Burkholderia cepacia* | Bacterial brown spot of cymbidium |
| *Burkholderia gladioli* pv. *gladioli* | Neck rot of gladiolus |
| *Burkholderia glumae* | Bacterial grain rot of rice |
| *Burkholderia plantarii* | Bacterial seedling blight of rice |
| *Clavibacter michiganensis* subsp. *michiganensis* | Bacterial canker of tomato |
| *Clavibacter michiganensis* subsp. *sepedonicus* | Ring rot of potato |
| *Clostridium* spp. | Slimy rot of potato |
| *Curtobacterium flaccumfaciens* | Bacterial canker of onion |
| *Erwinia amylovora* | Fire blight of pear |
| *Erwinia ananas* | Bacterial palea browning of rice |
| *Erwinia carotovora* subsp. *atroseptica* | Black leg of potato |
| *Erwinia carotovora* subsp. *carotovora* | Bacterial soft rot of vegetables |
| *Erwinia chrysanthemi* | Bacterial seedling blight of taro |
| *Erwinia chrysanthemi* pv. *zeae* | Bacterial foot rot of rice |
| *Erwinia herbicola* pv. *millettiae* | Bacterial gall of wisteria |
| *Pseudomonas cichorii* | Bacterial spot of chrysanthemum |
| *Pseudomonas corrugate* | Pith necrosis of tomato |
| *Pseudomonas fuscovaginae* | Sheath brown rot of rice |
| *Pseudomonas marginalis* pv. *marginalis* | Soft rot of cabbage |
| *Pseudomonas rubrisubalbicans* | Mottled stripe of sugar cane |
| *Pseudomonas syringe* pv. *aptata* | Bacterial blight of sugar beet |
| *Pseudomonas syringae* pv. *atropurpurea* | Halo blight of ryegrass |
| *Pseudomonas syringae* pv. *castaneae* | Bacterial canker of chestnut |
| *Pseudomonas syringae* pv. *glycinea* | Bacterial blight of soybean |
| *Pseudomonas syringae* pv. *lachrymans* | Bacterial spot of cucumber |
| *Pseudomonas syringae* pv. *maculicola* | Bacterial black spot of cabbage |
| *Pseudomonas syringae* pv. *mori* | Bacterial blight of mulberry |
| *Pseudomonas syringae* pv. *morsprunorum* | Bacterial canker of plums |
| *Pseuedomonas syringae* pv. *oryzae* | Halo blight of rice |
| *Pseudomonas syringae* pv. *phaseolicola* | Halo blight of kidney bean |
| *Pseudomonas syringae* pv. *pisi* | Bacterial blight of garden pea |
| *Pseudomonas syringae* pv. *sesame* | Bacterial spot of sesame |
| *Pseudomonas syringae* pv. *striafaciens* | Bacterial stripe blight of oats |
| *Pseudomonas syringae* pv. *syringae* | Bacterial brown spot of small red bead |
| *Pseudomonas syringae* pv. *tabaci* | Wild fire of tobacco |
| *Pseudomonas syringae* pv. *theae* | Bacterial shoot blight of tea |
| *Pseudomonas syringae* pv. *tomato* | Bacterial leaf spot of tomato |
| *Pseudomonas viridiflava* | Bacterial brown spot of kidney bean |
| *Ralstonia solanacearum* | Bacterial wilt |
| *Rathayibacter rathayi* | Bacterial head blight of orchardgrass |
| *Streptomyces scabies* | Common scab of potato |
| *Streptomyces ipomoea* | Soil rot of sweet potato |
| *Xanthomonas albilineans* | White streak of sugar cane |
| *Xanthomonas campestris* pv. *cerealis* | Bacterial streak of rye |
| *Xanthomonas campestris* pv. *campestris* | Black rot |
| *Xanthomonas campestris* pv. *citri* | Canker of citrus |
| *Xanthomonas campestris* pv. *cucurbitae* | Bacterial brown spot of cucumber |
| *Xanthomonas campestris* pv. *glycines* | Bacterial pastule of soybean |
| *Xanthomonas campestris* pv. *incanae* | Black rot of stock |
| *Xanthomonas campestris* pv. *malvacearum* | Angular leaf spot of cotton |
| *Xanthomonas campestris* pv. *Mangiferaeindicae* | Bacterial canker of mango |
| *Xanthomonas campestris* pv. *mellea* | Wisconsin bacterial leaf spot of tobacco |
| *Xanthamonas campestris* pv. *nigromaculans* | Bacterial spot of great burdock |
| *Xanthomonas campestris* pv*. phaseoli* | Bacterial pastule of kidney bean |
| *Xanthomonas campestris* pv. *pisi* | Bacterial stem-rot of kidney |
| *Xanthomonas campestris* pv. *pruni* | Bacterial shot hole of peach |
| *Xanthomonas campestris* pv*. raphani Xanthomonas campestris* pv. *raphani* | Bacterial spot of Japanese radish |
| *Xanthomonas campestris* pv*. ricini* | Bacterial spot of castor-oil |
| *Xanthomonas campestris* pv. *theicola* | Canker of tea |
| *Xanthomonas campestris* pv. *translucens* | Bacterial blight of orchardgrass |
| *Xanthomonas campestris* pv. *vesicatoria* | Bacterial spot of tomato |
| *Xanthomonas oryzae* pv. *oryzae* | Bacterial leaf blight of rice |

Specific examples of plant pathogenic bacteria include *Acidovorax avenae* subsp. *avenae, Agrobacterium tumefaciens, Burkholderia caryophylli, Burkholderia glumae, Erwinia carotovora* subsp. *carotovora, Ralstonia solanacearum,* and *Xanthomonas campestris* pv. *campestris.*

Similarly, examples of plant pathogenic fungi include, but are not limited to, fungi listed in Table 2 below: In Table 2, the fungi listed in the left column can cause the corresponding diseases listed in the right column.

**Table 2:**

| Fungal name | Disease name |
|---|---|
| *Fusarium oxysporum* | Fusarium wilt |
| *Vertiucillium dahliae* | Verticillium wilt |

Examples of animal pathogenic bacteria include, but are not limited to, *Burkholderia cepacia* that is a pathogen of a pulmonary infectious disease accompanied by human cystic fibrosis.

When a microorganism is a bacterium or fungus, basic medium components can include a nitrogen source, a buffer substance, and a mineral substance. More specifically, when a microorganism is a bacterium, basic medium components include preferably an ammonium salt, a substance constituting phosphoric acid buffer, a magnesium salt, and a calcium salt. More preferably, basic medium components include ammonium chloride, disodium hydrogen phosphate, potassium dihydrogen phosphate, magnesium sulfate, and calcium chloride. The contents (concentrations) thereof can be adequately adjusted. However, for example, the composition of Table 3 below is preferable. In addition to a medium having the composition of Table 3. for example, a medium having a composition obtained by removing glucose (sugar) from the composition of the M9 minimal medium (Miller, JH, 1972) can be used.

**Table 3:**

| Component name | Content |
|---|---|
| Na₂HPO₄ | 3 g |
| KH₂PO₄ | 3 g |
| NH₄Cl | 1 g |
| MgSO₄ | 0.25 g |
| CaCl₂ | 67 mg |
| Water | 1 L |

When a microorganism is a fungus, basic medium components include preferably nitrate, a substance constituting phosphoric acid buffer, a magnesium salt, and an iron salt. More preferably, basic medium components include sodium nitrate, potassium chloride, dipotassium hydrogen phosphate, magnesium sulfate, and iron sulfide. Unlike bacteria, fungi cannot metabolize ammonium salts. In addition, a mineral substance required for fungal proliferation is not calcium. Therefore, when a microorganism is a fungus, basic medium components include nitrate and iron instead of an ammonium salt and calcium. The contents (concentrations) thereof can be adequately adjusted. However, for example, the composition of Table 4 below is preferable.

**Table 4:**

| Component name | Content |
|---|---|
| KCl | 1 g |
| K₂HPO₄ | 1 g |
| NaNO₃ | 2 g |
| MgSO₄/7H₂O | 0.25 g |
| FeSO₄/7H₂O | 10 mg |
| Water | 1 L |

When a carbon source assimilated by microorganism is selected based on biological information about the microorganism, limited search is preferably conducted in advance by the method described below so as to search for the carbon source that should be selected.

Any carbon source, crystal violet (antibiotic) and agar are mixed with basic medium components so as to prepare an agar medium. A soil suspension (100 µl) prepared by suspending of soil for plant cultivation (1 g) in water (100 ml) is smeared (repeatedly 3 times) on the medium, followed by culture at 30°C for 5 days. The numbers of colonies of filamentous bacterial cells and other bacterial cells that are formed on the medium are counted. Each counted value is represented as a relative value (%) determined based on the number of colonies formed on a "carbon source-free" medium (100%). If the degree of colony formation is less than 30 (for filamentous bacterial cells) and is less than 60 (for bacterial cells), the carbon source is selected as a carbon source having high growth inhibitory effects. The smaller value indicates the smaller number of contaminating germs (filamentous bacteria and other bacteria) that assimilate the carbon source.

Table 5 below lists carbon sources previously tested by the above method.

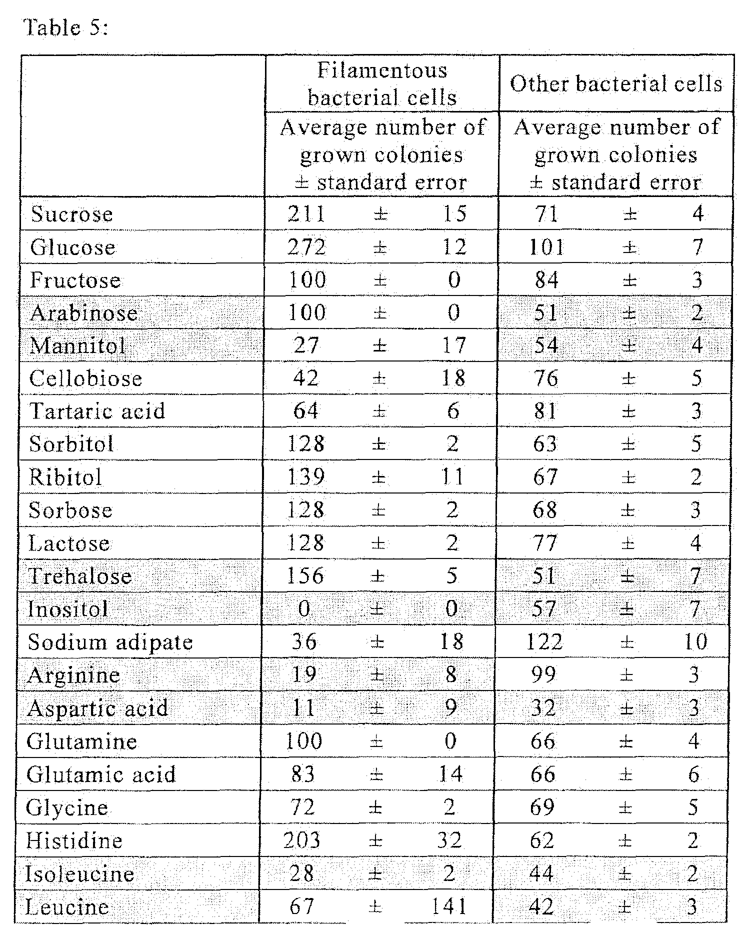

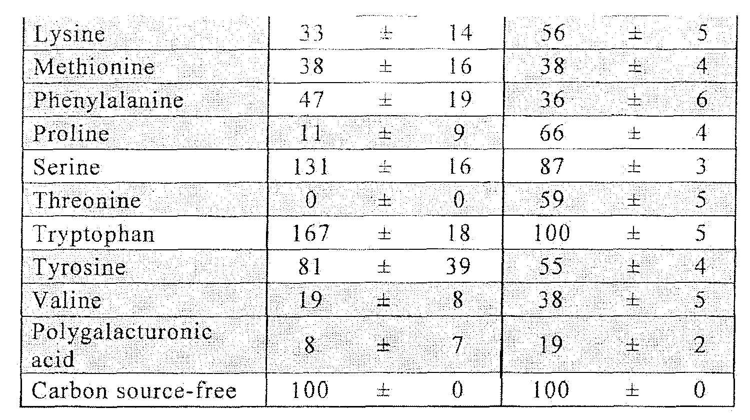

As shown in Table 5, carbon sources that are less likely to be assimilated by contaminating germs in soil are arabinose, mannitol, trehalose, inositol, arginine, aspartic acid, isoleucine, leucine, lysine, methionine, phenylalanine, proline, threonine, tyrosine, valine, and polygalacturonic acid. Note that in addition to these carbon sources, it is preferable to add glutamic acid that serves as either a carbon source or a nitrogen source and is a useful starting substance for various forms of biosynthesis. Therefore, when a carbon source assimilated by a microorganism is selected based on biological information about the microorganism, it is preferable for the carbon source to be selected from the group consisting of arabinose, mannitol, trehalose, inositol, arginine, aspartic acid, isoleucine, leucine, lysine, methionine, phenylalanine, proline, threonine, glutamic acid, tyrosine, valine, and palygalacturonic acid in order to cause the microorganism to predominantly proliferate.

For example, a carbon source assimilated by *Acidovorax avenae* subsp. *avenae* can be selected based on the carbon metabolism information about *Acidovorox avetiae* subsp. *avenae* by the method comprising the following steps of: accessing the KEGG database (http://www.genome.jp/kegg/); clicking "KEGG PATHWAY;" selecting "MODULE" as a subject of "Search;" inputting an arbitrary carbon source name as a search term into the column next to "for;" pressing the button labeled "Go" to initiate search; clicking "Pathway map" in the displayed list to transfer to the "Pathway map" page; selecting *"Acidovorax avenae"* from the pull-down bar of the bacterial name list; selecting a carbon source (i.e., an "extracellular" carbon source) for each selected metabolism pathway; and selecting a carbon source linked to "Glycolysis" from among the extracellular carbon, sources.

In addition, a medium is prepared by mixing the selected carbon source and basic medium components. Then, *Acidovorax avenae* subsp. *avenae* and contaminating germs are smeared thereon. Thereafter, a carbon source that is highly likely to be assimilated by *Acidovorax avenae* subsp. *avenue* but less likely to be assimilated by contaminating germs is selected. Thus, the optimal carbon source appropriate for a selective medium can be selected from among the selected carbon sources.

In the KEGG database, the relevant metabolism pathway for each bacterium is shown in the pathway page. Fig. 1 shows an example of such metabolism pathway. In Fig. 1, D-Mannitol (one type of sugar) is marked with a red circle. The "[1.1.1.1 1] " box drawn next to the red circle denotes an enzyme that degrades D-Mannitol into D-Fructose (written next to D-Mannitol). The presence of this box means that the enzyme can convert D-Mannitol into D-Fructose. This box is linked to the "Glycolysis" box shown at the lower right in Fig. 1. Linkage to "Glycolysis" means that ATP synthesis necessary for the growth of an organism can be induced, thereby allowing the organism to grow. If linkage from "D-Mannitol" to "Glycolysis" is achieved for an arbitrary bacterium with the use of the KEGG database in the above manner, it can be determined that the bacterium can use D-Mannitol. If no such linkage is achieved, it can be determined that the bacterium cannot use D-Mannitol to grow. Also, each of the above carbon sources other than D-Mannitol can be determined as a carbon source used for a medium by the above procedure.

In addition, the antibiotic used herein is preferably an antibiotic to which a target microorganism (but no other microorganisms) has resistance. It is more preferably a commonly available antibiotic. Further preferably, it is an antibiotic selected from the group consisting of ampicillin, chloramphenicol, novobiocin, cetrimide, polymyxin, carbenicillin, ticarcillin, penicillin, neomycin, tyrothricin, streptomycin, and vancomycin.

For example, an antibiotic to which *Acidovorax avenue* subsp. *avenue* has resistance can be selected based on the gene information about *Acidovorax avenae* subsp. *avenae* by a method comprising the following steps of: accessing the NCBI database (http://www.ncbi.nlm.nih.gov/); selecting "Nucleotide" as a subject of "Search;" inputting, as search terms, *Acidovorax avenae,* [ORGN], and an arbitrary antibiotic name in such order into the column next to "for;" pressing the button labeled "Go" to initiate search; and searching for the relevant antibiotic-resistant gene or the relevant antibiotic synthesis gene. At such time, if *Acidovorax avenae* subsp. *avenue* does not have the relevant antibiotic-resistant gene or the relevant antibiotic synthesis gene, no (zero) search result is obtained. Then, an antibiotic having either one of or both of the genes is selected as an antibiotic to be added to a selective medium. In addition, it is preferable to confirm based on references or the like whether or not the gene searched for corresponds to the relevant antibiotic-resistant gene or the relevant antibiotic synthesis gene.

As an antibiotic-resistant gene, for example, a gene encoding a protein that binds to an antibiotic so as to inactivate the antibiotic can be used. Specific examples of an antibiotic-resistant gene that can be used include: the Beta-lactamase gene resistant to carbenicillin; the tauD gene resistant to ticarcillin; the penicillin-binding protein IC gene resistant to penicillin; the strB gene resistant to streptomycin; the bme2 gene and the sanA gene resistant to vancomycin; the p-lactamase gene resistant to ampicillin; the arnA gene resistant to polymyxin; the chloramphenicol-acetylating enzyme gene resistant to chloramphenicol; and the gene encoding a homolog of a gene product of any of the above examples.

As an antibiotic synthesis gene, for example, a gene encoding a protein involved in synthesis of an antibiotic can be used. Specific examples of an antibiotic synthesis gene that can be used include: a neomycin phosphoric acid group transfer enzyme gene for neomycin (the neo gene); a tyrothricin synthesis enzyme gene for tyrothricin (the tycC gene); an extracellular cetrimide transfer pump gene for cetrimide (the sugE gene); a novobiocin efflux pump gene for novobiocin (the MdtA gene, the MdtB gene, or the MdtC gene); a novobiocin, synthesis enzyme gene (the aspC gene); and a gene encoding a homolog of a gene product of any of the above examples.

Components such as carbon sources and/or antibiotics selected based on biological information about a microorganism are mixed with basic medium components. A commonly knows mixing method involving, for example, manual mixing or mechanical mixing using a mixer or the like can be used without limitation. Upon mixing, it is possible to adequately determine the order of adding the components and the basic medium components described above. However, it is common to add structurally stable compounds in advance and then add structurally unstable compounds. Specific examples of a selective medium obtained by the above method include, but are not limited to, selective media with the compositions described in the Examples.

A selective medium obtained by the production method of the present invention may be a complete synthetic medium containing components such as carbon sources and antibiotics selected based on biological information about a microorganism and substances contained in or added to basic medium components such as a nitrogen source, a buffer substance, a mineral substance, a vitamin, a stain, and an antibiotic, all of which are chemically synthesized compounds. A complete synthetic medium is more advantageous than a semisynthetic medium or a natural medium containing natural components in that temperature or pH changes can be readily predicted, that there are fewer production lot differences, and that compounds used for a complete synthetic medium are widely commercially available. Therefore, a selective medium obtained by the production method of the present invention is preferably a complete synthetic medium.

The present invention encompasses a selective medium obtained by the production method of the present invention. Preferred examples of the selective medium of the present invention include, but are not limited to: a selective medium for *Burkholcleria glurnae* containing at least arabinose as a carbon source, one, two, three, four, or five type(s) of antibiotic(s) selected from the group consisting of ampicillin, chloramphenicol, novobiocin, cetrimide, and polymyxin, and basic medium components; a selective medium for *Acidovorax avenae* subsp. *avenae* containing at least mannitol as a carbon source, one, two, three, or four type(s) of antibiotic(s) selected from the group consisting of carbenicillin, ticarcillin, penicillzn, and neomycin, and basic medium components; a selective medium for *Agrobacterium tumefaciens* containing at least mannitol as a carbon source, one, two, or three type(s) of antibiotic(s) selected from the group consisting of tyrothricin, cetrimide, and vancomycin, and basic medium components; a selective medium for *Burkholderia caryophylli* containing at least sorbitol as a carbon source, one, two, three, or four type(s) of antibiotic(s) selected from the group consisting of tyrothricin, ampicillin, chloramphenicol, and polymyxin, and basic medium components; a selective medium for *Erwinia carotovara* subsp. *carotovora* containing at least pectic acid as a carbon source, one, two, three, or four type(s) of antibiotic(s) selected from the group consisting of neomycin, novobiocin, cetrimide, and vancomycin, and basic medium components; a selective medium for *Ralstonia solanacearum* containing L-glutamic acid as a carbon source, one, two, three, four, or five type(s) of antibiotic(s) selected from the group consisting of polymyxin, chloramphenicol, ticarcillin, carbenicillin, and vancomycin, and basic medium components; a selective medium for *Xanthomonas campestris* pv. *campestris* containing at least mannitol as a carbon source, one, two, or three type(s) of antibiotic(s) selected from the group consisting of novobiocin, neomycin, and vancomycin, and basic medium components; and a selective medium for *Burkholderia cepacia* containing arabinose as a carbon source, one or two type(s) of antibiotic(s) selected from the group consisting of streptomycin and ampicillin, and basic medium components. In addition, the larger the number of types of antibiotics added, the more preferable the media.

The selective medium of the present invention can be provided in the form of a commonly known medium without particular limitation. For example, it can be provided in the form of a liquid medium, a solid medium, or a medium-impregnated support. A commonly known method for producing a liquid medium can be used without limitation to produce the liquid selective medium of the present invention. For example, the liquid selective medium of the present invention can be produced by dissolving components such as a carbon source and an antibiotic selected based on biological information about a microorganism and basic medium components in a liquid (and preferably water). A commonly known method for producing a solid medium can be used without limitation to produce the solid selective medium of the present invention. For example, the solid selective medium of the present invention can be produced by dissolving components such as such as a carbon source and an antibiotic selected based on biological information about a microorganism, basic medium components, and a coagulable substance (and preferably agar) in a liquid (and preferably water) and solidifying the resulting solution.

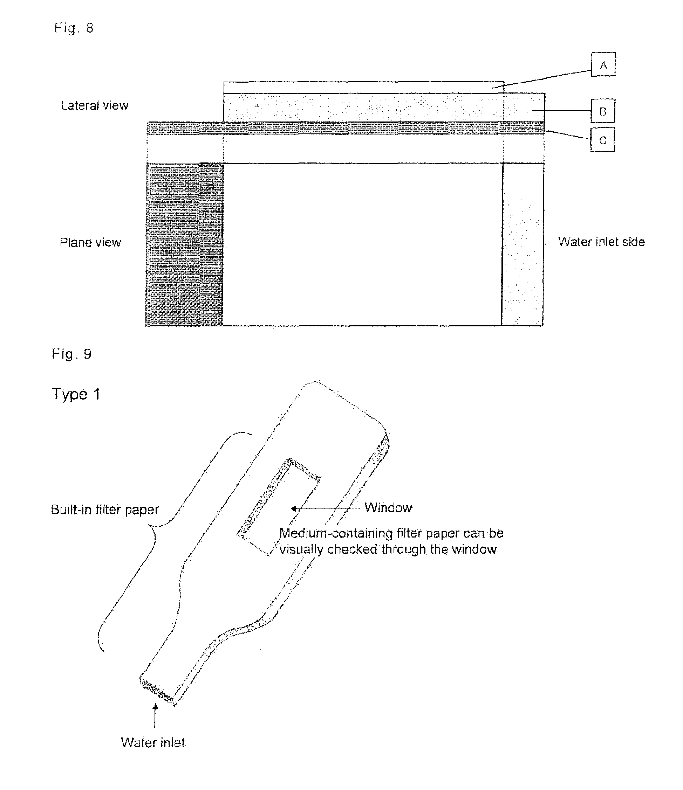

The selective medium in the form of a medium-impregnated support of the present invention is obtained by immersing or coasting a support made of paper, filter paper, fabric, a membrane, or the like with a liquid or solid selective medium to spread the medium into the support. Fig. 8 shows a preferable example of the selective medium of the present invention in the form of a medium-impregnated support. In Fig. 8, the layer "A" is an adhesive tape layer formed with a material such as paper, fabric, or shrink film having transparency, air permeability, and dry-proof/moisture-retaining properties. Also, the layer can be formed with a combination of the above material containing no adhesive components and double-faced adhesive tape. In Fig. 8, the layer "B" is a layer formed with a material such as paper, filter paper, fabric, or a membrane used for retaining components that constitute the medium. In Fig. 8, the layer "C" is a support (paper mat) layer formed with a material such as polyester, polypropylene, or polystyrene having waterproof properties. When a test solution is added dropwise to the exposed portion of the layer "B" of the selective medium shown in Fig. 8 on the water absorption side, it results in spontaneous diffusion of the solution into the layer "B." Then, if a microorganism to be selected is present in the solution, proliferation of the microorganism takes place. If it is not present therein, proliferation of the microorganism does not take place. A stain that causes staining when the microorganism is incorporated into the layer "B" shown in Fig. 8 is mixed with the solution. Thus, formation of colonies of the microorganism can be visually confirmed when proliferation of the microorganism takes place. The layer "B" shown in Fig. 8 may partially or entirely contain components selected based on biological information about a microorganism and basic medium components. For instance, a portion of the layer "B" shown in Fig. 8 can be determined to be a detection unit containing components selected based on biological information about a microorganism and basic medium components. In addition, a different portion of the layer "B" can be determined to be a control unit containing components that allow microorganisms other than the microorganism to proliferate.

The present invention encompasses a kit for detecting a microorganism that comprises the selective medium of the present invention. The kit of the present invention can be in the form of any conventionally known kit without limitation. However, it is preferably in a stick-type or liquid-type kit. The kit of the present invention can be used to confirm the presence or absence of a target microorganism in a sample. The kit of the present invention may comprise an equipment for applying a specimen comprising microorganisms to the selective medium of the present invention, in addition to the selective medium of the present invention.

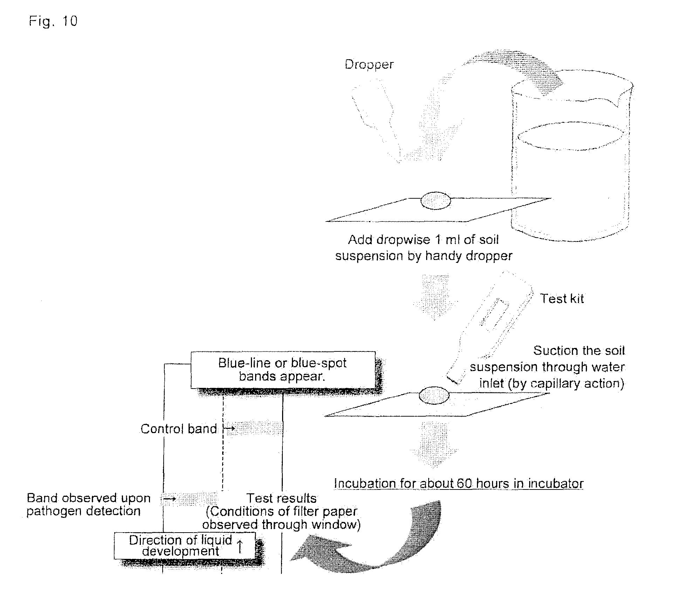

Fig. 9 shows a preferable example of the stick-type kit of the present invention. The kit of the present invention shown in Fig. 9 includes the selective medium of the present invention in the form of a medium-impregnated support and a corrosion-resistant stick-type member (rod) housing the selective medium and having a detection window through which a portion of the selective medium can be visually checked and a water inlet (opening). For example, the kit shown in Fig. 9 can be used in the following manner. A portion of a soil suspension is introduced on a hydrophobic membrane formed with paraffin or the like and suctioned via the water inlet into the selective medium by capillary action, following incubation for an appropriate time period at a temperature adequate for microorganism growth. Accordingly, colonies formed as a result of proliferation of a microorganism (i.e., a target microorganism) and colonies formed as a result of proliferation of other contaminating germs contained in soil (i.e., control microorganisms) can be confirmed through the detection window (Fig. 10).

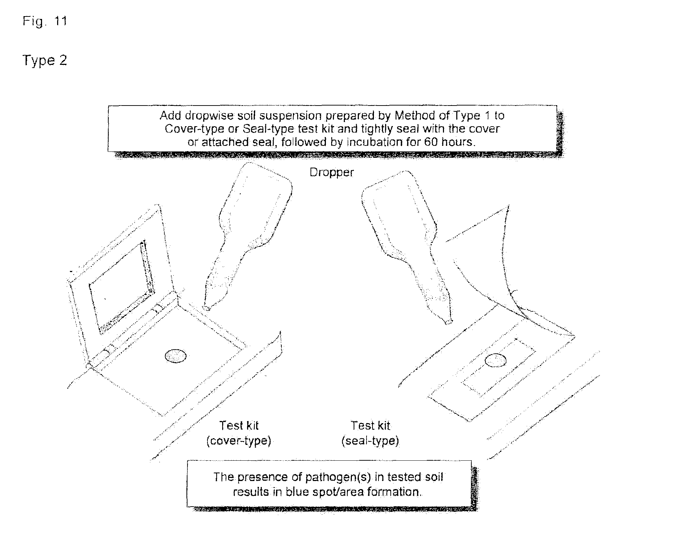

Another preferable example of the stick-type kit of the present invention can be the cover-type kit or seal-type kit in a stick shape shown in Fig. 11. Such kit partially contains the selective medium of the present invention. A specimen containing microorganisms is added dropwise to the selective medium, the kit is tightly closed with the cover or attached seal, and culture is carried out under conditions appropriate for the microorganism. Accordingly, colonies formed as a result of proliferation of the microorganisms can be confirmed.

For example, the liquid-type kit of the present invention comprises a container with a cap such as an Eppendorf tube into which the liquid selective medium of the present invention has been added. The liquid-type kit of the present invention can be used in the manner described below.

A sample suspension is prepared by mixing seeds or soil with a solution such as water or buffer and, if necessary, performing ultrasonication. A portion of the prepared sample suspension is added to the kit of the present invention, followed by incubation for 24 hours under conditions adequate for proliferation of a bacterium to be selected. As a result of incubation for 24 hours, contaminating germs die and the bacterium to be selected is exclusively allowed to proliferate. A reagent that stains only viable bacterial cells (ChemChrome V23) is added to the kit of the present invention subjected to incubation, followed by count with the use of a flow cytometer. Note that ChemChrome V23 absorbs laser light at 480 nm due to the esterase activity of the bacterium and is converted into a substance that emits excitation light at 525 nm. The obtained value would be equivalent to or exceed 100 cfu/ml only for a sample containing the bacterium to be selected. According to this method, it becomes possible to automatically examine many seed and soil samples with high sensitivity by nondestructive testing. The principles of flow cytometers are described as follows: a sample solution obtained by subjecting a bacterium in a bacterial suspension to fluorescent, staining is introduced into an apparatus; individual bacterial cells linearly flow in a flow cell of the apparatus; the flow is partially irradiated with a blue laser to detect fluorescence emitted from the bacterial cells; and the number of bacterial cells that have passed through the flow cell is counted. According to the techniques reported in the past, fluorescence staining is carried out using specific fluorescent antibodies or fluorescent DNA probes, followed by flow cytometry using a flow cytometer. However, background fluorescence emission due to a non-specific reaction is detected. As a result, the detection sensitivity is as low as 10⁵ cfu/ml. Therefore, such techniques are operationally complicated.

In the above method, when BTB is used instead of ChemChrome V23, bacterial proliferation can be visually confirmed based on the color change of the liquid in the kit, making it possible to avoid the use of a flow cytometer. For example, if 1 ml of the selective medium contains a bacterium at approximately 100 cfu, the liquid color changes from green to yellow after culture for 24 hours.

The present invention is hereafter described in greater detail with reference to the following examples, although the present invention is not limited thereto.

### Examples

### Example 1

### Method for producing selective medium for Acidovorax avenae subsp. avenae

### (1) Selection of carbon sources

Genome sequencing of *Acidovorax avenue* subsp. *avrenae* is still in progress. Thus, genomic information about *Aciciovorax avenae* subsp. *citrulli* (of the same species) was used. A limited search of carbon sources was conducted (Table 5). Based on the results, the following were subjected to primary screening: arabinose, mannitol, trehalose, inositol, arginine, aspartic acid, isoleucine, leucine, lysine, methionine, phenylalanine, proline, threonine, tyrosine, valine, and polygalacturonic acid.

The procedure carried out herein comprises the following steps of: accessing the KEGG database (http://www.genome.jp/kegg/); clicking "KEGG PATHWAY;" selecting "MODULE" as a subject of "Search;" inputting the name of any one of the above carbon sources subjected to primary screening as a search term into the column next to "for;" pressing the button labeled "Go" to initiate search; clicking "Pathway map" in the displayed list to transfer to the "Pathway map" page; selecting "*Acidovorax avenae*" from the pull-down bar of the bacterial name list; identifying a carbon source (i.e:, an "extracellular" carbon source) for each selected metabolism pathway; and selecting a carbon source linked to "Glycolysis." As a result, *Acidovorax avenae* subsp. *avenae* was found to have a mannitol-degrading enzyme gene. Therefore, mannitol was selected as the carbon source to be predominantly assimilated by *Acidovorax avenae* subsp. *avenae.*

### (2) Selection of antibiotics

The procedure carried out herein comprises the following steps of: accessing the NCBI database (http://www.ncbi.nlm.nih.gov/); selecting "Nucleotide" as a subject of "Search;" inputting, as search terms, *Acidovorax avenae,* [ORGN], and the name of a broad-spectrum antibiotic (i.e. ampicillin, streptomycin, chloramphenicol, novobiocin, cetrimide, polymyxin, carbenicillin, ticarcillin, penicillin, neomycin, tyrothricin, or vancomycin) in such order into a column next to "for;" pressing the button labeled "Go" to initiate search; and searching for the relevant antibiotic-resistant gene or the relevant antibiotic synthesis gene for each antibiotic. As a result, carbenicillin, ticarcillin, penicillin, and neomycin were selected as the antibiotics to which *Acidovorax avenae* subsp. *avenae* has resistance.

### (3) Basic medium components

The basic medium components used herein are listed in Table 6 with reference to the M9 minimal medium.

**Table 6:**

| Component name | Content |
|---|---|
| Na₂HPO₄ | 3 g |
| KH₂PO₄ | 3 g |
| NH₄Cl | 1 g |
| MgSO₄ | 0.25 g |
| CaCl₂ | 67 mg |
| Water | 1 L |

It was found that molybdenum is required for metabolism of mannitol in the mannitol metabolism pathway. Therefore, Na₂MoO₄/2H₂O was added. In addition, cycloheximide, which is a eukaryotic protein synthesis inhibitor and thus inhibits fungal growth, was added. Further, crystal violet, which is a stain capable of sterilizing gram-positive bacteria, was added.

### (4) Selective medium composition

Table 7 shows the composition of a selective medium (containing the above components) prepared in the form of a solid medium with the addition of agar.

**Table 7:**

| Component name | Content |
|---|---|
| Mannitol | 2 g |
| Basic medium components | 1 L |
| Na₂MoO₄/2H₂O | 25 mg |
| Cycloheximide | 50 mg |
| Crystal violet | 3 ppm |
| Carbenicillin | 10 mg |
| Ticarcillin | 10 mg |
| Penicillin | 10 mg |
| Neomycin | 10 mg |
| Agar | 20 g |

### (5) Evaluation of medium

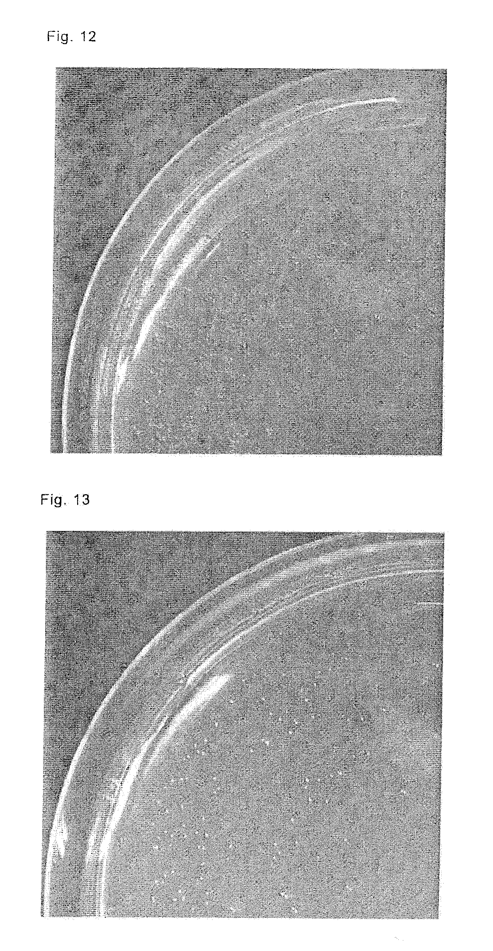

Selectivity in terms of proliferation of *Acidovorax avenae* subsp. *avenae* was evaluated using a selective medium having the composition of Table 7 (Fig. 12) and a previously reported medium having the composition of Table 8 (Fig. 1).

**Table 8:**

| Component name | Content |
|---|---|
| Diammoruum adipate | 10 g |
| KH₂PO₄ | 0.5 g |
| Na₂HPO₄/12H₂O | 2.0 g |
| (NH₄)₂SO₄ | 2.0 g |
| MgSO₄/7H₂O | 29 mg |
| CaCl₂/2H₂O | 67 mg |
| Yeast extract | 10 mg |
| Na₂MoO₄/2H₂O | 25 mg |
| BTB | 12.5 mg |
| Agar | 20 g |
| Distilled water | 1000 ml |
| Ampicillin | 20 mg |
| Phenethicillin potassium | 100 mg |
| Novobiocin | 2mg |
| Cycloheximide | 25 mg |

As is apparent from Figs. 1 and 12, *Acidovorax avenae* subsp. *avenae* was found to have more predominantly proliferated in the medium having the composition of Table 7 than in the medium having the composition of Table 8.

### Example 2

### Method for producing selective medium for Agrobacterium tumefaciens

### (1) Selection of carbon sources

Genome sequencing of *Agrobacterium tumefaciens* is still in progress. However, there are databases containing enormous quantities of genetic information about *Agrobacterium tumefaciens.* A limited search of carbon sources was conducted (Table 5). Based on the results, the following were subjected to primary screening: arabinose, mannitol, trehalose, inositol, arginine, aspartic acid, isoleucine, leucine, lysine, methionine, phenylalanine, proline, threonine, tyrosine, valine, and polygalacturonic acid.

The procedure carried out herein comprises the following steps of: accessing the KEGG database (http://www.genome.jp/kegg/); clicking "KEGG PATHWAY;" selecting "NODULE" as a subject of "search;" inputting the name of any one of the above carbon sources subjected to primary screening as a search term into the column next to "for;" pressing the button labeled "Go" to initiate search; selecting "*Agrobacterium tumefaciens*" from the pull-down bar of the bacterial name list; identifying a carbon source (i.e., an "extracellular" carbon source) for each selected metabolism pathway; and selecting a carbon source linked to "Glycolysis." As a result, *Agrobacterium tumefaciens* was found to have a mannitol-degrading enzyme gene. Therefore, mannitol was selected as the carbon source to be predominantly assimilated by *Agrobacterium tumefaciens.*

### (2) Selection of antibiotics

The procedure carried out herein comprises the following steps of: accessing the NCBI database (http://www.ncbi.nlm.nih.gov/); selecting "Nucleotide" as a subject of "Search;" inputting, as search terms, *Agrobacterium tumefaciens,* [ORGN], and the name of an antibiotic (i.e., ampicillin, streptomycin, chloramphenicol, novobiocin, cetrimide, polymyxin, carbenicillin, ticarcillin, penicillin, neomycin, tyrothricin, or vancomycin) in such order into a column next to "for;" pressing the button labeled "Go" to initiate search; and searching for the relevant antibiotic-resistant gene or the relevant antibiotic synthesis gene for each antibiotic. As a result, streptomycin, tyrothricin, cetrimide, and vancomycin were selected as the antibiotics to which *Agrobacterium tumefaciens* has resistance.

### (3) Basic medium components

The basic medium components used herein are listed in Table 6 with reference to the M9 minimal medium.

In addition, cycloheximide, which is a eukaryotic protein synthesis inhibitor and thus inhibits fungal growth, was added. Further, crystal violet, which is a stain capable of sterilizing gram-positive bacteria, was added.

### (4) Selective medium composition

Table 9 shows the composition of a selective medium (containing the above components) prepared in the form of a solid medium with the addition of agar.

**Table 9:**

| Component name | Content |
|---|---|
| Mannitol | 2 g |
| Basic medium components | 1 L |
| Cycloheximide | 50 mg |
| Crystal violet | 3 ppm |
| Streptomycin | 10 mg |
| Tyrothricin | 10 mg |
| Cetrimide | 10 mg |
| Vancamycin | 10 mg |
| Agar | 20 g |

### (5) Evaluation of medium

Selectivity in terms of proliferation of *Agrobacterium tumefaciens* was evaluated using a selective medium having the composition of Table 9 (Fig. 13) and a previously reported medium having the composition shown Table 10 (Fig. 2).

**Table 10:**

| Component | Content |
|---|---|
| Mannitol | 15 g |
| LiCl | 6 g |
| Na₂HPO₄ | 5 g |
| K₂HPO₄ | 2 g |
| MgSO₄ | 2 g |
| Ca(NO₃)₂ | 20 mg |
| Agar | 20 g |
| Distilled water | 1000 ml |

As is apparent from Figs. 2 and 13, *Agrobacterium tumefaciens* was found to have more predominantly proliferated in the medium having the composition of Table 9 than in the medium having the composition of Table 10.

### Example 3

### Method for producing selective medium for Burkholderia caryophylli

### (1) Selection of carbon sources

Genome sequencing of *Burkholderta caryophylli* is still in progress. However, there are databases containing enormous quantities of genetic information about *Burkholderia caryophylli.* A limited search of carbon sources was conducted (Table 5). Based on the results, the following were subjected to primary screening: arabinose, mannitol, trehalose, inositol, arginine, aspartic acid, isoleucine, leucine, lysine, methionine, phenylalanine, proline, threonine, tyrosine, valine, and polygalacturonic acid.

The procedure carried out herein comprises the following steps of: accessing the KEGG database (http://www.genome.jp/kegg/); clicking "KEGG PATHWAY;" selecting "MODULE" as a subject of "Search;" inputting the name of any one of the above carbon sources subjected to primary screening as a search term into the column next to "for;" pressing the button labeled "Go" to initiate search; selecting "*Burkholderia caryophylli* " from the pull-down bar of the bacterial name list; identifying a carbon source (i.e., an "extracellular" carbon source) for each selected metabolism pathway; and selecting a carbon source linked to "Glycolysis." As a result, *Burkholderia caryophylli* was found to have a sorbitol-degrading enzyme gene. Therefore, sorbitol was selected as the carbon source to be predominantly assimilated by *Burkholderia caryophylli.*

### (2) Selection of antibiotics

The procedure carried out herein comprises the following steps of: accessing the NCBI database (http://www.ncbi.nlm.nih.gov/); selecting "Nucleotide" as a subject of "search;" inputting, as search terms, *Burkholderia caryophylli,* [ORGN], and the name of an antibiotic (i.e., ampicillin, streptomycin, chloramphenicol, novobiocin, cetrimide, polymyxin, carbenicillin, ticarcillin, penicillin, neomycin, tyrothricin, or vancomycin) in such order into a column next to "for;" pressing the button labeled "Go" to initiate search; and searching for the relevant antibiotic-resistant gene or the relevant antibiotic synthesis gene for each antibiotic. As a result, tyrothricin, ampicillin, chloramphenicol, and polymyxin were selected as the antibiotics to which *Burkholderia caryophylli* has resistance.

### (3) Basic medium components

The basic medium components used herein are listed in Table 6 with reference to the M9 minimal medium. In addition, cycloheximide, which is a eukaryotic protein synthesis inhibitor and thus inhibits fungal growth, was added. Further, crystal violet, which is a stain capable of sterilizing gram-positive bacteria, was added. In order to increase the colony formation rate by increasing the amino acid synthesis rate, arginine was added.

### (4) Selective medium composition

Table 11 shows the composition of a selective medium (containing the above components) prepared in the form of a solid medium with the addition of agar.

**Table 11:**

| Component name | Content |
|---|---|
| Sorbitol | 2 g |
| Basic medium components | 1 L |
| | 50 mg |
| Cycloheximide Crystal violet | 5 mg |
| Tyrothricin | 10 mg |
| Ampicillin | 50 mg |
| Chloramphenicol | 10 mg |
| Polymyxin | 50 mg |
| Agar | 20 g |
| Arginine | 1 g |

### (5) Evaluation of medium

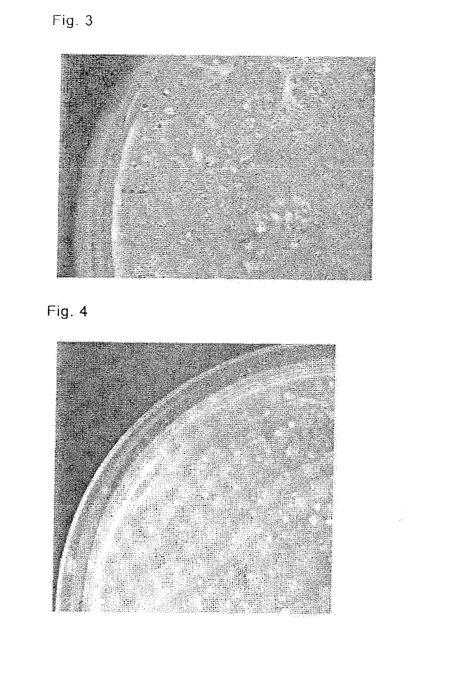

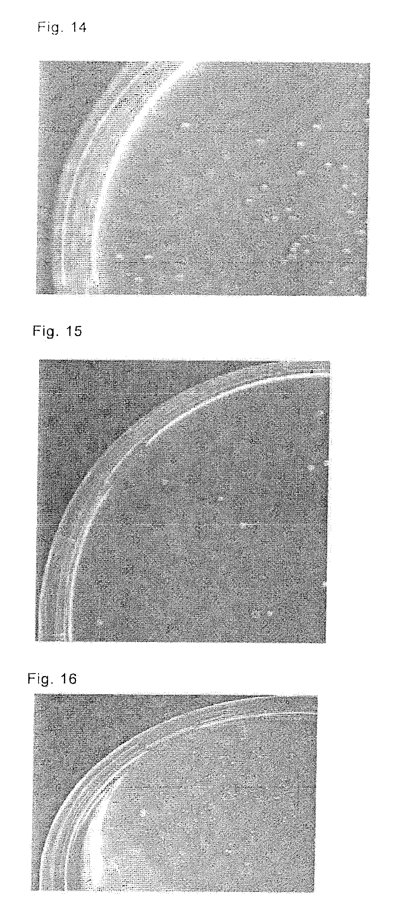

Selectivity in terms of proliferation of *Burkholderia caryophylli* was evaluated using a selective medium having the composition of Table 11 (Fig. 14) and a previously reported medium having the composition shown Table 12 (Fig. 3).

**Table 12:**

| Component name | Content |
|---|---|
| Arabinose | 2 g |
| (NH₄)₂SO₄ | 0.79 g |
| KH₂PO₄ | 1 g |
| MgSO₄ | 0.5 g |
| KCl | 0.2 g |
| Deoxycholate | 2 g |
| Agar | 20 g |

As is apparent from Figs. 3 and 14, *Burkholderia catyophylli* was found to have more predominantly proliferated in the medium having the composition of Table 12 than in the medium having the composition of Table 11.

### Example 4

### Method for producing selective medium for Burkholderia glumae

### (1) Selection of carbon sources

Genome sequencing of *Burkholderia glumae* is still in progress. However, there are databases containing enormous quantities of genetic information about *Burkholderia gluma.* A limited search of carbon sources was conducted (Table 5). Based on the results, the following were subjected to primary screening: arabinose, mannitol, trehalose, inositol, arginine, aspartic acid, isoleucine, leucine, lysine, methionine, phenylalanine, proline, threonine, tyrosine, valine, and polygalacturonic acid.

The procedure carried out herein comprises the following steps of: accessing the KEGG database (http://www.genome.jp/kegg/); clicking "KEGG PATHWAY;" selecting "MODULE" as a subject of "Search;" inputting the name of any one of the above carbon sources subjected to primary screening as a search term into the column next to "for;" pressing the button labeled "Go" to initiate search; selecting "*Burkholderia glumae*" from the pull-down bar of the bacterial name list; identifying a carbon source (i.e., an "extracellular" carbon source) for each selected metabolism pathway; and selecting a carbon source linked to "Glycolysis." As a result, *Burkholderia glumae* was found to have an arabinose transporter gene (the AraG gene). Therefore, arabinose was selected as the carbon source to be predominantly assimilated by *Burkholderia glumae.*

### (2) Selection of antibiotics

The procedure carried out herein comprises the following steps of: accessing the NCBI database (http://www.ncbi.nlm.nih.gov/); selecting "Nucleotide" as a subject of "Search;" inputting, as search terms, *Btirkholderia glumae,* [ORGN], and the name of an antibiotic (i.e., ampicillin, streptomycin, chloramphenicol, novobiocin, cetrimide, polymyxin, carbenicillin, ticarcillin, penicillin, neomycin, tyrothricin, or vancomycin) in such order into a column next to "for;" pressing the button labeled "Go" to initiate search; and searching for the relevant antibiotic-resistant gene or the relevant antibiotic synthesis gene for each antibiotic. As a result, ampicillin, chloramphenicol, novobiocin, cetrimide, and polymyxin were selected as the antibiotics to which *Burkholderia glumae* has resistance.

### (3) Basic medium components

The basic medium components used herein are listed in Table 6 with reference to the M9 minimal medium. In addition, cycloheximide, which is a eukaryotic protein synthesis inhibitor and thus inhibits fungal growth was added. Further, crystal violet, which is a stain capable of sterilizing gram-positive bacteria, was added. In order to increase the colony formation rate by increasing the amino acid synthesis rate, arginine was added.

### (4) Selective medium composition

Table 1 3 shows the composition of a selective medium (containing the above components) prepared in the form of a solid medium with the addition of agar.

**Table 13:**

| component name | Content |
|---|---|
| Arabinose | 2 g |
| Basic medium components | 1 L |
| Cycloheximide | 50 mg |
| Crystal violet | 5 mg |
| Ampicillin | 50 mg |
| Chloramphenicol | 10 mg |
| Novobiocin | 1 mg |
| Cetrimide | 10 mg |
| Polymyxin | 50 mg |
| Arginine | 1 g |
| Agar | 20 g |

### (5) Evaluation of medium

Selectivity in terms of proliferation of *Burkholderia glumae* was evaluated using a selective medium having the composition of Table 13 (Fig. 15) and a previously reported medium having the composition shown Table 14 (Fig. 4).

**Table 14:**

| Component name | Content |
|---|---|
| Inositol | 4 g |
| Yeast extract | 2 g |
| Peptone | I g |
| Cetrimide | 10 mg |
| Chloramphenicol | 10 mg |
| Novobiocin | 1 mg |
| Daconil® | |
| (Tetrachloroisophthalonitrile) | 100 mg |
| Agar | 20 g |

As is apparent from Figs. 4 and 15, *Burkholderia glumae* was found to have more predominantly proliferated in the medium having the composition of Table 14 than in the medium having the composition of Table 13.

### Example 5

### method for producing selective medium for Erwinia carotovora subsp._carotovora

### (1) Selection of carbon sources

Genome sequencing of *Erwinia carotovora* subsp. *carotovora* is still in progress. However, there are databases containing enormous quantities of genetic information about *Erwinia carotovora* subsp. *carotovora.* A limited search of carbon sources was conducted (Table 5). Based on the results, the following were subjected to primary screening: arabinose, mannitol, trehalose, inositol, arginine, aspartic acid, isoleucine, leucine, lysine, methionine, phenylalanine, proline, threonine, tyrosine, valine, and polygalacturonic acid.

The procedure carried out herein comprises the following steps of: accessing the KEGG database (http://www.genome.jp/kegg/); clicking "KEGG PATHWAY;" selecting "MODULE" as a subject of "search;" inputting the name of any one of the above carbon sources subjected to primary screening as a search term into the column next to "for;" pressing the button labeled "Go" to initiate search; selecting *"Erwinia carotovora* subsp. *carotovora"* from the pull-down bar of the bacterial name list; identifying a carbon source (i.e., an "extracellular" carbon source) for each selected metabolism pathway; and selecting a carbon source linked to "Glycolysis." As a result, *Erwinia carotovora subsp. carotovora* was found to have a pcctie-acid-degrading enzyme (pectic acid lyase 3) gene. Therefore, pectic acid was selected as the carbon source to be predominantly assimilated by *Erwinia carotovora* subsp. *carotovora.*

### (2) Selection of antibiotics

The procedure carried out herein comprises the following steps of: accessing the NCBI database (http://www.ncbi.nlm.nih.gov/); selecting "Nucleotide" as a subject of "Search;" inputting, as search terms, *Erwinia carotovora* subsp. *carotovora,* [ORGN], and the name of an antibiotic (i.e., ampicillin, streptomycin, chloramphenicol, novobiocin, cetrimide, polymyxin, carbenicillin, ticarcillin, penicillin, neomycin, tyrothricin, or vancomycin) in such order into a column next to "for;" pressing the button labeled "Go" to initiate search; and searching for the relevant antibiotic-resistant gene or the relevant antibiotic synthesis gene for each antibiotic. As a result, neomycin, novobiocin, cetrimide, and vancomycin were selected as the antibiotics to which *Erwinia carotovora* subsp. *carotovora* has resistance.

### (3) Basic medium components

The basic medium components used herein are listed in Table 6 with reference to the M9 minimal medium. In addition, cycloheximide, which is a eukaryotic protein synthesis inhibitor and thus inhibits fungal growth, was added. Further, crystal violet, which is a stain capable of sterilizing gram-positive bacteria, was added.

### (4) Selective medium composition

Table 15 shows the composition of a selective medium (containing the above components) prepared in the form of a solid medium with the addition of agar.

**Table 15:**

| Component name | Content |
|---|---|
| Pectic acid | 2 g |
| Basic medium components | 1 L |
| Cycloheximide | 50 mg |
| Crystal violet | 5 mg |
| Neomycin | 10 mg |
| Novobiocin | 10 mg |
| Cetrimide | 10 mg |
| Vancomycin | 10 mg |
| Agar | 20 g |

### (5) Evaluation of medium

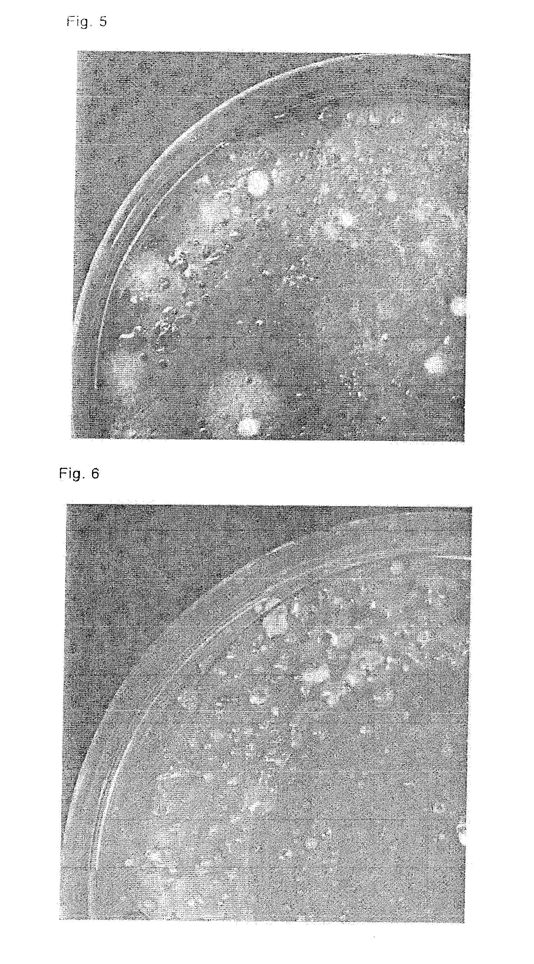

Selectivity in terms of proliferation of *Erwinia carotovora* subsp. *carotovora* was evaluated using a selective medium having the composition of Table 15 (Fig. 16) and a previously reported medium having the composition shown Table 16 (Fig. 5).

**Table 16:**

| Component name | Content |
|---|---|
| Lactose | 10 g |
| Meat extract | 10 g |
| Peptone | 10 g |
| NaCl | 5 g |
| Crystal violet | 5 mg |
| BTB | 80 mg |
| Agar | 20 g |
| Distilled water | 1 L |

As is apparent from Figs. 15 and 16, *Erwinia caratovora* subsp. *carotovora* was found to have more predominantly proliferated in the medium having the composition of Table 15 than in the medium having the composition of Table 16.

### Example 6

### Method for producing selective medium for Ralstonia solanacearum

### (1) Selection of carbon sources

Genome sequencing of *Ralstonia solanacearum* has been completed. A limited search of carbon sources was conducted (Table 5). Based on the results, the following were subjected to primary screening: arabinose, mannitol, trehalose, inositol, arginine, aspartic acid, isoleucine, leucine, lysine, methionine, phenylalanine, proline, threonine, tyrosine, valine, and polygalacturonic acid.

The procedure carried out herein comprises the following steps of: accessing the KEGG database (http://www.genome.jp/kegg/); clicking "KEGG PATHWAY;" selecting "NODULE" as a subject of "Search;" inputting the name of any one of the above carbon sources subjected to primary screening as a search term into the column next to "for;" pressing the button labeled "Go" to initiate search; selecting *"Ralstonia solanacearum"* from the pull-down bar of the bacterial name list; identifying a carbon source (i.e., an "extracellular" carbon source) for each selected metabolism pathway; and selecting a carbon source linked to "Glycolysis." As a result, *Ralstonia solanacearum* was found use L-glutamic acid as a starting material for synthesis of a carbon compound or a nitrogen compound. Therefore, L-glutamic acid was selected as the carbon source to be predominantly assimilated by *Ralstonia solanacearum.*

### (2) Selection of antibiotics

The procedure carried out herein comprises the following steps of: accessing the NCBI database (http://www.ncbi.nim.nih.gov/); selecting "Nucleotide" as a subject of "Search;" inputting, as search terms, *Ralstonia solanacearum*, [GRGN], and the name of an antibiotic (i.e., ampicillin, streptomycin, chloramphenicol, novobiocin, cetrimide, polymyxin, carbenicillin, ticarcillin, penicillin, neomycin, tyrothricin, and vancomycin) in such order into a column next to "for;" pressing the button labeled "Go" to initiate search; and searching for the relevant antibiotic-resistant gene or the relevant antibiotic synthesis gene for each antibiotic. As a result, polymyxin, chloramphenicol, ticarcillin, carbenicillin, and vancomycin were selected as the antibiotics to which *Ralstonia solanacearum* has resistance.

### (3) Basic medium components

The basic medium components used herein are listed in Table 6 with reference to the M9 minimal medium. In addition, cycloheximide, which is a eukaryotic protein synthesis inhibitor and thus inhibits fungal growth, was added. Further, crystal violet, which is a stain capable of sterilizing gram-positive bacteria, was added.

### (4) Selective medium composition

Table 17 shows the composition of a selective medium (containing the above components) prepared in the form of a solid medium with the addition of agar.

**Table 17:**

| Component name | Content |
|---|---|
| L-glutamic acid | 1 g |
| Basic medium components | 1 L |
| Cycloheximide | 50 mg |
| Crystal violet | 3 mg |
| Polymyxin | 50 mg |
| Chloramphenicol | 5 mg |
| Ticarcillin | 10 mg |
| Carbenicillin | 10 mg |
| Vancomycin | 10 mg |
| Agar | 20 g |

### (5) Evaluation of medium

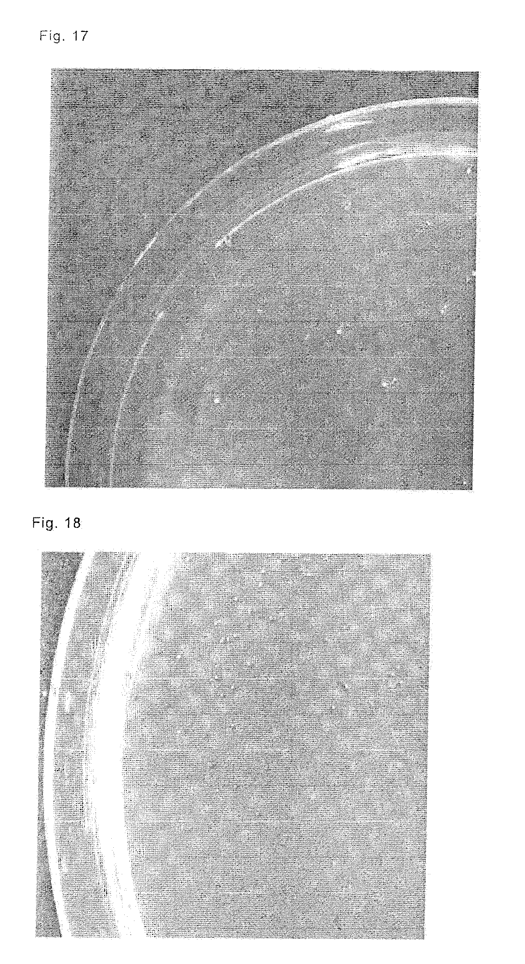

Selectivity in terms of proliferation of *Ralstonia solanacearum* was evaluated using a selective medium having the composition of Table 17 (Fig. 17) and a previously reported medium having the composition shown Table 18 (Fig. 6).

**Table 18:**

| Component name | Content |
|---|---|
| Potato decoction | 1 L |
| Cycloheximide | 50 mg |
| Crystal violet | 5 mg |
| Polymyxin | 50 mg |
| Chloramphenicol | 10 mg |
| Agar | 20 g |

As is apparent from Figs. 6 and 17, *Ralstonia solanacearum* was found to have more predominantly proliferated in the medium having the composition of Table 17 than in the medium having the composition of Table 18.

### Example 7

### Method for producing selective medium for Xanthomonas campestris pv. campestris

### (1) Selection of carbon sources

Genome sequencing of *Xanthomonas campestris* pv. *vesicatoria* which is closely related to *Xanthomonas campestris pv. campestris* has been completed. A limited search of carbon sources was conducted (Table 5). Based on the results, the following were subjected to primary screening: arabinose, mannitol, trehalose, inositol, arginine, aspartic acid, isoleucine, leucine, lysine, methionine, phenylalanine, proline, threonine, tyrosine, valine, and polygalacturonic acid.

The procedure carried out herein comprises the following steps of: accessing the KEGS database (http://www.genome.jp/kegg/); clicking "KEGG PATHWAY;" selecting "MODULE" as a subject of "search;" inputting the name of any one of the above carbon sources subjected to primary screening as a search term into the column next to "for;" pressing the button labeled "Go" to initiate search; selecting *"Xanthomonas campestris* pv. *campestris"* from the pull-down bar of the bacterial name list; identifying a carbon source (i.e., an "extracellular" carbon source) for each selected metabolism pathway; and selecting a carbon source linked to '"Glycolysis." As a result, *Xanthomonas campestris* pv. *campestris* was found to have a mannitol-degrading enzyme gene. Therefore, mannitol was selected as the carbon source to be predominantly assimilated by *Xanthomonas campestris* pv. *campestris.*

### (2) Selection of antibiotics

The procedure carried out herein comprises the following steps of: accessing the NCBI database (http://www.ncbi.nlm.nih.gov/); selecting "Nucleotide" as a subject of "Search;" inputting, as search terms, *Xanthomonas campestris* pv. *campestris,* [ORGN], and the name of an antibiotic (i.e., ampicillin, streptomycin, chloramphenicol, novobiocin, cetrimide, polymyxin, carbenicillin, ticarcillin, penicillin, neomycin, tyrothricin, or vancomycin) in such order into a column next to "for;" pressing the button labeled "Go" to initiate search; and searching for the relevant antibiotic-resistant gene or the relevant antibiotic synthesis gene for each antibiotic. As a result, novobiocin, vancomycin, and neomycin were selected as the antibiotics to which *Xanthomonas campestris* pv. *campestris* has resistance.

### (3) Basic medium components

The basic medium components used herein are listed in Table 6 with reference to the M9 minimal medium. It was found that iron and molybdenum are required for metabolism of mannitol in the mannitol metabolism pathway. Therefore, Fe-EDTA and Na₂MoO₄/2H₂O were added. In addition, cycloheximide, which is a cukaryotic protein synthesis inhibitor and thus inhibits fungal growth, was added. Further, crystal violet, which is a stain capable of sterilizing gram-positive bacteria, was added.

### (4) Selective medium composition

Table 19 shows the composition of a selective medium (containing the above components) prepared in the form of a solid medium with the addition of agar.

**Table 19:**

| component name | Content |
|---|---|
| Mannitol | 2 g |
| Basic medium components | 1 L |
| Na₂MoO₄/2H₂O | 25 mg |
| Fe-EDTA | 10 mg |
| Cycloheximide | 50 mg |
| Crystal violet | 3 mg |
| Novobiocin | 10 mg |
| Vancomycin | 10 mg |
| Neomycin | 10 mg |
| Agar | 20 g |

### (5) Evaluation of medium

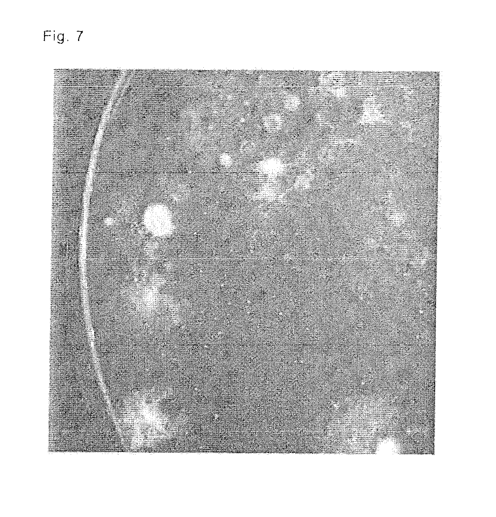

Selectivity in terms of proliferation of *Xanthomonas campestris* pv. *camprestris* was evaluated using a selective medium having the composition of Table 19 (Fig. 18) and a previously reported medium having the composition shown Table 20 (Fig. 7).

**Table 20:**

| Component name | Content |
|---|---|
| Potato decoction | 1 L |
| L-methionine | 2 g |
| KH₂PO₄ | 1 g |
| NaH₂PO₄ | 2.6 g |
| NH₄Cl | 1 g |
| NaCl | 2 g |
| MgSO₄ | 0.2 g |
| CaC1₂ | 10 mg |
| Na₂MoO₄/2H₂O | 25 mg |
| Fe-EDTA | 10 mg |
| ZnSO₄/7H₂O | 10 mg |
| CuSO₄/H₂O | 10 mg |
| MnSO₄/H₂O | 10 mg |
| Methyl violet 2B | 10 mg |
| Methyl green | 20 mg |
| Agar | 20 g |

As is apparent from Figs. 7 and 18, *Xanthomonas campestris* pv. *campestris* was found to have more predominantly proliferated in the medium having the composition of Table 19 than in the medium having the composition of Table 20.

### Example 8

### Detection of pathogenic bacteria using a liquid selective medium

### (1) Seeds

Seeds were added to basic medium components (900 ul), followed by ultrasonication for 10 minutes. The thus prepared suspension contained microorganisms that had been originally contained in the seeds. A selective medium (100 ul) prepared by replacing the basic medium components described in Example 1 or 4 with water was added to the suspension, provided that the seeds were removed (or not removed) from the suspension. Shake culture was conducted at 37°C and 2000 rpm/min for 24 hours. Thus, a sample solution was obtained. A portion of the sample solution (100 ul) was collected and a fluorescent reagent (ChemChrome V23; 1 ul) was added thereto, and the volume was adjusted to 1 ml, followed by analysis using a flow cytometer.

### (2) Soil

Soil (1 g) was added to basic medium components (10 µl). The resultant was vortexed for 10 minutes so as to prepare a suspension containing microorganisms that had been originally contained in seeds. The suspension was allowed to stand still for 10 minutes. The obtained supernatant (100 ul) was centrifuged at 10000 rpm for 10 minutes and the supernatant was discarded. Basic medium components (900 µl) and a selective medium (100 µl) obtained by replacing the basic medium components described in Example 3 or 6 with water were added thereto. Shake culture was conducted at 37°C and 180 rpm/min for 24 hours. Thus, a sample solution was obtained. A portion of the sample solution (100 µl) was collected and a fluorescent reagent (ChemChrome V23; 1 ul) was added thereto, and the volume was adjusted to 1 ml, followed by analysis using a flow cytometer. According to this method, 0.01 g of soil would be actually examined. For instance, if a bacterium is contained in contaminated soil at 10³ cfu/l g of soil, 10 cfu of the bacterium would be contained in a liquid selective medium for selective culture.

### (3) Detection sensitivity

When seeds are examined, the detection sensitivity is 1 cfu of a pathogen /1 seed. When soil is examined, it is 10000 cfu of a pathogen/1 g of soil. Note that if a nutritive medium is used, detection can be carried out with a detection sensitivity of 1000 cfu/1 g of soil.

### (4) Detection of Burkholderia glumae

Ten test seeds suspected to be infected were used as test samples. As a result of flow cytometer analysis by the method described in (1), the value obtained by CyFlow exceeded 100 for Test seeds 2, 7, and 10. These seeds were determined to be infected seeds (Table 21). In this Example, identification was carried out by PCR using a primer set comprising glu-FW (SEQ ID NO: 1 in the Sequence Listing) and glu-RV (SEQ ID NO: 2 in the Sequence Listing). As a result of examination by the above method, seed germination was not affected.

**Table 21:**

| Sample | FCM level (cfu/ml) | Specific PCR results |
|---|---|---|
| Alcohol sterilize, seed | 20 | - |
| Healthy seed 1 | 25 | |
| Healthy seed 2 | 15 | - |
| Healthy seed 3 | 15 | - |
| Test seed 1 | 35 | - |
| Test seed 2 | 1495 | + |
| Test seed 3 | 15 | - |
| Test seed 4 | 20 | - |
| Test seed 5 | 5 | - |
| Test seed 6 | 10 | - |
| Test seed 7 | 420 | + |
| Test seed 8 | 10 | - |
| Test seed 9 | 15 | - |
| Test seed 10 | 665 | + |

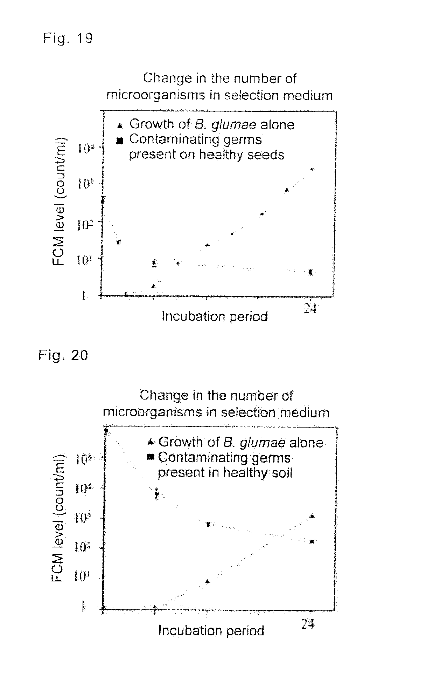

As a result of examination of the change in the number of microorganisms during culture for 24 hours, it was found that 24-hour culture caused proliferation of a pathogen with an increase from 1 cfu to approximately 3000 cfu (Fig. 19). However, as a result of actual determination, the level exceeding 3000 cfu/ml was not obtained from any of the contaminated seeds. This is probably because the proliferation preparation time (lag phase) was necessary for proliferation of *B. glumae* present on seeds.

The value displayed by a flow cytometer (corresponding to the background noise level of the flow cytometer) was found to be approximately 10 also for healthy healthy seeds. However, in fact, no colony formation was caused even by smearing the solution obtained after 24-hour culture of healthy seeds on a nutritive medium. Accordingly, the number of contaminating germs obtained after 24-hour selective culture became 0 for healthy seeds.

### (5) Detection of Acidovorax avenae

Ten test seeds suspected to be infected were used as test samples. As a result of flow cytometer analysis by the method described in (1), the value obtained by CyFlow exceeded 100 for all seeds. The seeds were determined to be infected seeds (Table 22). In this Example, identification was carried out by PCR using a primer set comprising Aaaf3 (SEQ ID NO: 3 in the Sequence Listing) and Aaar2 (SEQ ID NO: 4 in the Sequence Listing).

**Table 22:**

| Sample | FCM level (cfu/ml) | Specific PCR results |
|---|---|---|
| Alcohol-sterilized seed | 30 | - |
| Healthy seed 1 | 35 | - |
| Healthy seed 2 | 15 | - |
| Healthy seed 3 | 10 | - |
| Test seed 1 | 400 | + |
| Test seed 2 | 950 | + |
| Test seed 3 | 8930 | + |
| Test seed 4 | 970 | + |
| Test seed 5 | 940 | + |
| Test seed 6 | 500 | + |
| Test seed 7 | 840 | + |
| Test seed 8 | 4010 | + |
| Test seed 9 | 785 | + |
| Test seed 10 | 360 | + |

### (6) Detection of Burkholderia caryophylli

As a test sample, 0.01 g of soil was used. A sample solution prepared by the method described in (2) and a solution obtained as a 10-fold dilution of the sample solution were analyzed using a flow cytometer (Table 23). It is usually said that when a soil infectious germ is contained in soil at a level exceeding 10³ cfu/1 g of soil, it causes the onset of a disease. In this Experiment, the value obtained by CyFlow increased for contaminated soil with the level equivalent to or exceeding 4.56 x 10⁴ cfu/1 g of soil. In this case, it was possible to confirm contamination of soil based on the value. However, there were no obvious differences in terms of the value obtained by CyFlow between contaminated soil (4.56 x 10³ cfu/l g of soil) and healthy soil. When the liquid medium subjected to 24-hour selective culture was smeared on a nutritive medium without determination with the use of CyFlow, colony formation did not take place in healthy soil, while colony formation (approximately 200 cfu) took place in soil (4.56 x 10³ cfu/l g of Soil).

**Table 23:**

| Sample | Degree of contamination | Value obtained by CyFlow (cfu/ml) | Value obtained by CyFlow (cfu/ml) in 10-fold dilution |
|---|---|---|---|
| Autoclave-sterilized soil | - | 285 | 20 |
| Healthy soil | - | 370 | 20 |
| Artificially contaminated soil | 4.56 × 10³ cfu/lg of soil | 265 | 5 |
| | 4.56 × 10⁴ cfu/lg of soil | 1775 | 265 |
| | 4.56 × 10⁵ cfu/lg of soil | 3220 | 2705 |
| | 4,56 × 10⁶ cfu/lg of soil | 68400 | 12650 |
| | 4.56 × 10⁷ cfu/lg of soil | 703500 | 24300 |

In the case of healthy soil in Table 23, the value obtained by CyFlow was found to be 370. This corresponds to the background noise level. Therefore, no colony formation was observed even by smearing the soil on a nutritive medium. In the case of contaminated soil (4.56 × 10³ cfu/l g of soil), the value obtained by CyFlow was found to be 265. However, colony formation (approximately 200 cfu) was caused by smearing the soil on a nutritive medium. Thus, it was possible to distinguish contaminated soil from healthy soil. There were obvious differences in terms of the value obtained by CyFlow between contaminated soil (with the level equivalent to or exceeding 4.56 × 10⁴ cfu/l g of soil) and healthy soil.

As a result of examination of the change in the number of microorganisms during 24-hour culture, the value displayed by a flow cytometer (corresponding to the background noise level of the flow cytometer) was found to be approximately 300 also for healthy soil. However, in fact, no colony formation was caused even by smearing the solution obtained after 24-hour culture of healthy soil on a nutritive medium. Accordingly, the number of contaminating germs obtained after 24-hour selective culture became 0 for healthy soil (Fig. 20).

The value displayed by a flow cytometer (corresponding to the background noise level of the flow cytometer) was found to be approximately 10 also for healthy healthy seeds. However, in fact, no colony formation was caused even by smearing the solution obtained after 24-four culture of healthy seeds on a nutritive medium. Accordingly, the number of contaminating germs obtained after 24-hour selective culture became 0 for healthy seeds.

### (7) Detection of Ralstonia solanacearum

As a test sample, 0.01 g of soil was used. A sample solution prepared by the method described in (2) and a solution obtained as a 10-fold dilution of the sample solution were analyzed using a flow cytometer (Table 24).

**Table 24:**

| Sample | Degree of contamination | Value obtained by CyFlow (cfu/ml) | Value obtained by CyFlow (cfu/ml) of 10-fold dilution |
|---|---|---|---|
| Autoclave-sterilized soil | - | 425 | 15 |
| Healthy soil | - | 310 | 45 |
| Artificially contaminated soil | 1.52 × 10³ cfu/lg of soil | 455 | 50 |
| | 1.52 × 10⁴ cfu/lg of soil | 2340 | 180 |
| | 1.52 × 10⁵ cfu/lg of soil | 12545 | 450 |
| | 1.52 × 10⁶ cfu/lg of soil | 58755 | 8595 |
| | 1.52 × 10⁷ cfu/lg of soil | 145260 | 11450 |

In the case of healthy soil in Table 24, the value obtained by CyFlow was found to be 310. This corresponds to the background noise level. Therefore, no colony formation was observed even by smearing the soil on a nutritive medium. In the case of contaminated soil (1.52 × 10³ cfu/lg of soil), the value obtained by CyFlow was found to be 455. However, colony formation (approximately 400 cfu) was caused by smearing the soil on a nutritive medium. Thus, it was possible to distinguish contaminated soil from healthy soil. There were obvious differences in terms of the value obtained by CyFlow between contaminated soil (with the level equivalent to or exceeding 1.52 × 10⁴ cfu/lg of soil) and healthy soil.

### Example 9

### Detection of Burkholderia caryophylli with a system without flow cytometer

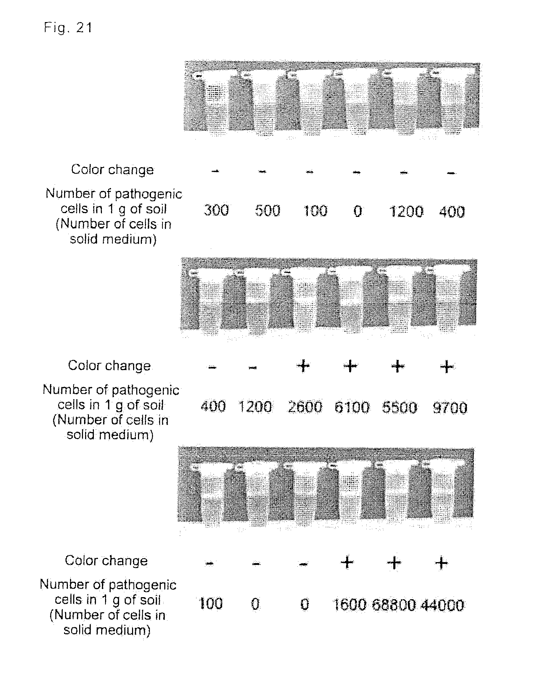

Soil (1 g) was suspended in distilled water (10 ml) and the obtained supernatant (100 µl) was used (provided that the supernatant corresponded to a 100-fold dilution of soil). The supernatant (100 µl), a 0.5% BTB solution (100 µl), basic medium components (800 ul), and a selective medium obtained by replacing the basic medium components described in Example 3 (100 µl) with water were mixed, followed by 24-hour culture. The occurrence or nonoccurrence of proliferation of *Burkholderia caryophylli* was confirmed based on the color change of the sample solution after culture (Fig. 21). At the same time, the number of *Burkholderia caryophylli* cells contained in soil (1 g) was determined using the selective medium described in Example 3. As a result, the color change was observed when the number of bacterial cells contained in soil (1 g) was found to be 1600 or more. Since the liquid selective medium contained a 100-fold dilution of soil in the above case, it is understood that the presence of bacterial cells (16 cfu) would cause color change.

### Example 10

### Method for producing selective medium for Burkholderia cepacia

### (1) Selection of carbon sources

Genome sequencing of *Burkholderia cepacia* has been substantially completed. Metabolism information about *Burkholderia cepacia* can be obtained from the KEGG and NCBI databases. A limited search of carbon sources was conducted (Table 5). Based on the results, the following were subjected to primary screening: arabinose, mannitol, trehalose, inositol, arginine, aspartic acid, isoleucine, leucine, lysine, methionine, phenylalanine, proline, threonine, tyrosine, valine, and polygalacturonic acid.

The procedure carried out herein comprises the following steps of. accessing the KEGG database (http://www.genome.jp/kegg/); clicking "KEGG PATHWAY;" selecting "MODULE" as a subject of "Search;" inputting the name of any one of the above carbon sources subjected to primary screening as a search term into the column next to "for;" pressing the button labeled "Go" to initiate search; selecting "*Burkholderia cepacia*" from the pull-down bar of the bacterial name list; identifying a carbon source (i.e., an "extracellular" carbon source) for each selected metabolism pathway; and selecting a carbon source linked to "Glycolysis." As a result, *Burkholderia ceuacia* was found to have an arabinose transporter gene (AraG). Therefore, arabinose was selected as the carbon source to be predominantly assimilated by *Burkholderia cepacia.*

### (2) Selection of antibiotics

The procedure carried out herein comprises the following steps of: accessing the NCBI database (http://www.ncbi.nlm.nih.gov/); selecting "Nucleotide" as a subject of "Search;" inputting, as search terms, *Burkholderia cepacia,* [ORGN], and the name of an antibiotic (i.e., ampicillin, streptomycin, chloramphenicol, novobiocin, cetrimide, polymyxin, carbenicillin, ticarcillin, penicillin, neomycin, tyrothricin, or vancomycin) in such order into a column next to "for;" pressing the button labeled "Go" to initiate search; and searching for the relevant antibiotic-resistant gene or the relevant antibiotic synthesis gene for each antibiotic. As a result, streptomycin and ampicillin were selected as the antibiotics to which *Burkholderia cepacia* has resistance,

### (3) Basic medium components

The basic medium components used herein are listed in Table 6 with reference to the M9 minimal medium. Cycloheximide, which is an eukaryotic protein synthesis-inhibiting agent and inhibits the fungal growth, was added thereto. Also, crystal violet, which is a stain and is capable of sterilizing gram-positive bacteria, was added thereto. Further, gentamicin was added thereto because *Burkholderia cepacia* has the gene encoding rpIF, which is a gentamicin-resistant complex protein L6 components.

### (4) Selective medium composition

Table 25 shows the composition of a selective medium (containing the above components) prepared in the form of a solid medium with the addition of agar.

**Table 25:**

| Component name | Content |
|---|---|
| Arabinose | 2 g |
| Basal medium | 1 L |
| Cycloheximide | 50 mg |
| Crystal violet | 5 mg |
| Streptomycin | 10 mg |
| Gentamicin | 10 mg |
| Ampicillin | 10 mg |
| Agar | 20 g |

### (5) Evaluation of medium

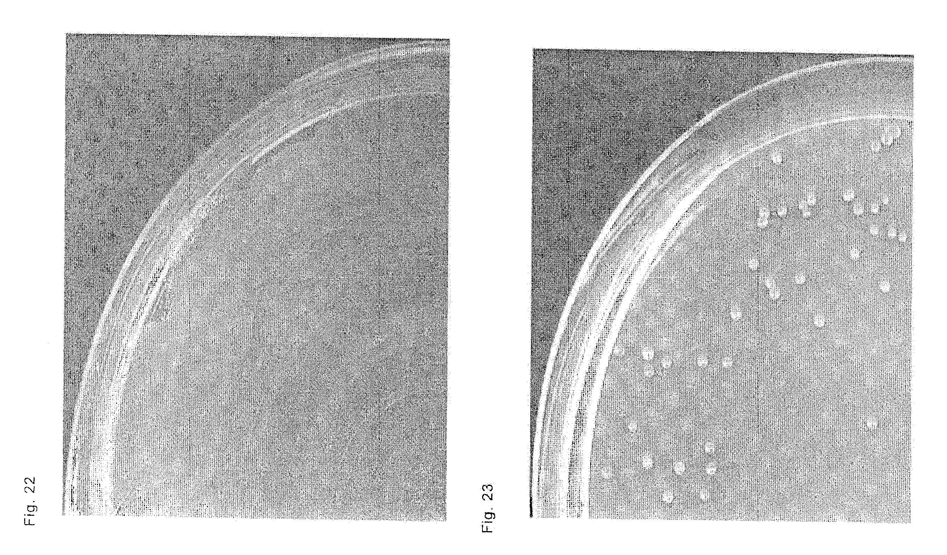

The selectivity in terms of *Burkholderia cepacia* proliferation was evaluated using a selective medium having the composition listed in Table 25 (Fig. 23) and a commercially available medium having the composition listed in Table 26 (*Burkholderia cepacia* agar base medium; OXOID; product code: CM0995)(Fig. 22).

**Table 26:**

| Component name | Content |
|---|---|
| Peptone | 5g |
| Yeast extract | 4 g |
| Sodium pyruvate | 7 g |
| Potassium dihydrogen phosphate | 4.4 g |
| Disodium hydrogen phosphate | 1.4 g |
| Bile salt | 1.5 g |
| Ammonium sulfate | I g |
| Magnesium sulfate | 0.2 g |
| Ammonium sulfate | 0.01 g |
| Phenol red | 0.02 g |
| Crystal violet | 0.001 g |
| Agar | 12 g |
| Pure water | 1 L |
| Polymyxin B | 150000 IU |
| Gentamicin | 5 mg |
| Ticarcillin | 100 mg |

As is apparent from Figs. 22 and 23, *Burkholderia cepacia* was found to have more predominantly proliferated in the medium having the composition of Table 25 than in the medium having the composition of Table 26.

## Claims

1. A method for producing a selective medium for a microorganism, which comprises the steps of: selecting at least one carbon source assimilable by a microorganism and/or at least one antibiotic to which the microorganism has resistance based on biological information about the microorganism; and mixing the selected carbon source and/or antibiotic with basic medium components.

2. A method for producing a selective medium for a microorganism, which comprises the steps of: selecting at least one carbon source assimilable by the microorganism and at least one antibiotic to which the microorganism has resistance based on biological information about the microorganism; and mixing the selected carbon source and antibiotic with basic medium components.

3. The production method according to claim 1 or 2, wherein the basic medium components comprise a nitrogen source, a buffer substance, and a mineral substance.

4. The production method according to any one of claims I to 3, wherein the microorganism is a bacterium or a fungus.

5. The production method according to claim 4, wherein the bacterium is *Acidovorax avenae* subsp. *avenae, Agrobacterium tumefaciens, Burkholderia caryophylli, Burkholderia glumae, Erwinia carotovora* subsp. *carotovora, Ralstonia solanacearum, Xanthomonas campestris* pv. *campestris,* or *Burkholderia cepacia.*

6. The production method according to any one of claims 1 to 5, wherein biological information based on which the carbon source is selected is information about carbon metabolism of the microorganism.

7. The production method according to any one of claims 1 to 6, wherein biological information based on which the antibiotic is selected is information about the gene of the microorganism.

8. The production method according to claim 7, wherein the gene is an antibiotic-resistant gene or an antibiotic synthesis gene.

9. The production method according to any one of claims 1 to 8, wherein at least one carbon source assimilable by the microorganism is at least one carbon source selected from the group consisting of arabinose, mannitol, trehalose, inositol, arginine, aspartic acid, isoleucine, leucine, lysine, methionine, phenylalanine, proline, threonine, glutamic acid, tyrosine, valine, and polygalacturonic acid.

10. The production method according to any one of claims 1 to 9, wherein the at least one antibiotic to which the microorganism has resistance is at least one antibiotic selected from the group consisting of ampicillin, chloramphenicol, novobiocin, cetrimide, polymyxin, carbenicillin, ticarcillin, penicillin, neomycin, tyrothricin, streptomycin, and vancomycin.

11. The production method according to any one of claims 1 to 10, wherein the selective medium for a microorganism is a complete synthetic medium.

12. A selective medium for a microorganism, which is produced by the production method according to any one of claims 1 to 11.

13. The selective medium according to claim 12, wherein the microorganism is *Burkholderia glumae,* arabinose is contained as the carbon source, and at least one antibiotic selected from the group consisting of ampicillin, chioramphenicol, novobiocin, cetrimide, and polymyxin is contained as the antibiotic.

14. The selective medium according to claim 12, wherein the microorganism is *Acidovorax avenae* subsp. *avenae,* mannitol is contained as the carbon source, and at least one antibiotic selected from the group consisting of carbenicillin, ticarcillin, penicillin, and neomycin is contained as the antibiotic.

15. The selective medium according to claim 12, wherein the microorganism is *Agrobacterium tumefaciens,* mannitol is contained as the carbon source, and at least one antibiotic selected from the group consisting of tyrothricin, cetrimide, and vancomycin is contained as the antibiotic.

16. The selective medium according to claim 12, wherein the microorganism is *Burkholderia caryophylli,* sorbitol is contained as the carbon source, and at least one antibiotic selected from the group consisting of tyrothricin, ampicillin, chloramphenicol, and polymyxin is contained as the antibiotic.

17. The selective medium according to claim 12, wherein the microorganism is *Erwinia carotovara* subsp. *carotovora,* pectic acid is contained as the carbon source, and at least one antibiotic selected from the group consisting of neomycin, novobiocin, cetrimide, and vancomycin is contained as the antibiotic.

18. The selective medium according to claim 12, wherein the microorganism is *Ralstonia solanacearum,* L-glutamic acid is contained as the carbon source, and at least one antibiotic selected from the group consisting of polymyxin, chloramphenicol, ticarcillin, carbenicillin, and vancomycin is contained as the antibiotic.

19. The selective medium according to claim 12, wherein the microorganism is *Xanthotnorcas carnpestris* pv. *campestris,* mannitol is contained as the carbon source, and at least one antibiotic selected from the group consisting of novobiocin, neomycin, and vancomycin is contained as the antibiotic.

20. The selective medium according to claim 12, wherein the microorganism is *Burkholderia cepacia,* arabinose is contained as the carbon source, and at least antibiotic selected from the group consisting of streptomycin and ampicillin is contained as the antibiotic.

21. The selective medium according to any one of claims 12 to 20, which is in the form of a liquid medium, a solid medium, or a medium-impregnated support.

22. A kit for detecting a microorganism, which comprises the selective medium according to any one of claims 12 to 21.

23. The kit according to claim 22, which is in the form of a stick-type kit or a liquid-type kit.


SEQUENCE LISTING
<110>  the University of Tokyo
<120>  method of manufacturing a medium for selection and use thereof
<130>  101050A
<160>  4
<170>  PatentIn version 3.4
<210>  1
<211>  18
<212>  DNA
<213>  Artificial sequence
<220>
<223>  Primer ; glu-FW
<400>  1
gaagtgtcgc cgatggag                                                    18
<210>  2
<211>  20
<212>  DNA
<213>  Artificial sequence
<220>
<223>  Primer ; glu-RV
<400>  2
ccttcaccga cagcacgcat                                                  20
<210>  3
<211>  20
<212>  DNA
<213>  Artificial sequence
<220>
<223>  Primer ; Aaaf3
<400>  3
gtcatcctcc accaaccaag                                                  20
<210>  4
<211>  23
<212>  DNA
<213>  Artificial sequence
<220>
<223>  Primer ; Aaar2
<400>  4
agaacaattc gtcattactg aac                                              23